# EUROPEAN PATENT SPECIFICATION

(11) **EP 2 688 863 B1**
(45) Date of publication and mention of the grant of the patent: **23.11.2016**
(21) Application number: 12710917.1
(22) Date of filing: 20.03.2012
(51) Int. Cl.: C07C 211/41, C07C 209/68

(54) **COMPOUNDS FOR USE IN IMAGING, DIAGNOSING AND/OR TREATMENT OF DISEASES OF THE CENTRAL NERVOUS SYSTEM**
VERBINDUNGEN ZUR VERWENDUNG BEI DER BILDGEBUNG, DIAGNOSE UND/ODER BEHANDLUNG VON ERKRANKUNGEN DES ZENTRALEN NERVENSYSTEMS
COMPOSÉ DESTINÉ À ÊTRE UTILISÉ EN IMAGERIE, DIAGNOSTIC ET/OU TRAITEMENT DE MALADIES DU SYSTÈME NERVEUX CENTRAL

(30) Priority: 23.03.2011 EP 11159427
(43) Date of publication of application: 29.01.2014
(73) Proprietor: Piramal Imaging SA, 1753 Matran (CH)
(72) Inventor: THIELE, Andrea, 13125 Berlin (DE); KETTSCHAU, Georg, 13187 Berlin (DE); HEINRICH, Tobias, 13507 Berlin (DE); LEHMANN, Lutz, 10115 Berlin (DE); HALLDIN, Christer, S-11332 Stockholm (SE); NAG, Sangram, S-16433 Kista Stockholm (SE); VARRONE, Andrea, 16738 Bromma (DE); GULYÁS, Balazs, S-17165 Solna Stockholm (SE)
(74) Representative: Kilger, Ute
(86) International application number: PCT/EP2012/054917
(87) International publication number: WO 2012/126913

(56) References cited:
- WO-A1-2011/024156
- WO-A2-2009/052970

## Description

### Field of the invention

This invention relates to novel compounds suitable for labelling by ¹⁸F and the corresponding ¹⁸F labelled compounds themselves, ¹⁹F-fluorinated analogues thereof and their use as reference standards, methods of preparing such compounds, compositions comprising such compounds, kits comprising such compounds or compositions and uses of such compounds, compositions or kits for diagnostic imaging by Positron Emission Tomography (PET).

### Background

Molecular imaging has the potential to detect disease, disease progression or therapeutic effectiveness earlier than most conventional methods in the fields of oncology, neurology and cardiology. Of the several promising molecular imaging technologies having been developed such as optical imaging, magnetic resonance imaging (MRI), single photon emission computed tomography (SPECT), and positron emission tomography (PET), PET is also of particular interest for drug development because of its high sensitivity and ability to provide quantitative and kinetic data.

Positron (β⁺) emitting isotopes include for example carbon, iodine, fluorine, nitrogen, and oxygen. These isotopes can replace their non-radioactive counterparts in target compounds to produce tracers for PET imaging that function biologically and are chemically identical to the original molecules, or can be attached to said counterparts to give close analogues of the respective parent effector molecules. Among these isotopes ¹⁸F is the most convenient labelling isotope due to its relatively long half life (110 min) which permits the preparation of diagnostic tracers and subsequent study of biochemical processes. In addition, its low ß⁺ energy (634 keV) is also advantageous.

The nucleophilic aromatic and aliphatic [¹⁸F]-fluoro-fluorination reaction is of great importance for [¹⁸F]-fluoro-labelled radiopharmaceuticals which are used as *in vivo* imaging agents targeting and visualizing diseases, e.g. solid tumours or diseases of brain. A very important technical goal in using [¹⁸F]-fluoro-labelled radiopharmaceuticals is the quick preparation and administration of the radioactive compound.

Monoamine oxidases (MAO, EC, 1.4.3.4) represent a distinct class of amine oxidases. Monoamine oxidases are present in two isoforms known as MAO-A and MAO-B (Med. Res. Rev. 1984, 4: 323-358). Crystal structures of MAO-A and MAO-B complexed by ligands have been reported (J. Med. Chem. 2004, 47: 1767-1774 and Proc. Nat. Acad. Sci. USA 2005, 102: 12684-12689).

In the human brain the presence of MAO-B predominates over MAO-A. Cerebral MAO-B levels increase with age and are further up-regulated in the brains of Alzheimer's disease (AD) patients mostly due to an increase of reactive astrocytes. As astrocyte activity and, consequently, the activity of the MAO-B system is up-regulated in neuroinflammatory processes, radiolabelled MAO-B inhibitors may serve as an imaging biomarker in neuroinflammation and neurodegeneration, including Alzheimer's disease.

Inhibitors that are selective for either MAO-A or MAO-B have been identified and investigated (e.g. J. Med. Chem. 2004, 47: 1767-1774 and Proc. Nat. Acad. Sci. USA, 2005, 102: 12684-12689).
Deprenyl (compound **A**), a MAO-B inhibitor (Biochem. Pharmacol. 1972, 5: 393-408) and clorgyline (**B**), a MAO-A inhibitor (Acta Psychiatr. Scand. Suppl. 1995, 386: 8-13), are potent monoamine oxidase inhibitors inducing irreversible inhibition of the enzymes. The (R)-isomer of deprenyl (Selegilin®, compound **(R)-A**) is a more potent inhibitor than the (S)-isomer (not shown).

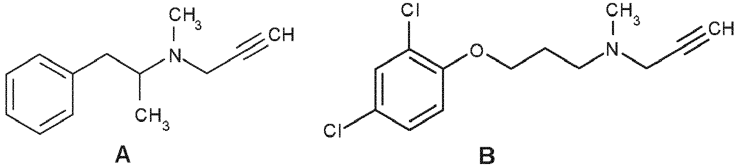

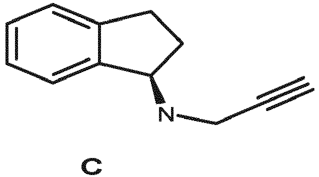

Another novel potent MAO-B inhibitor recently approved for use in treatment of Parkinson's disease is rasagiline (Azilect®, compound C) (Prog. Neurobiol. 2010, 92: 330-344).

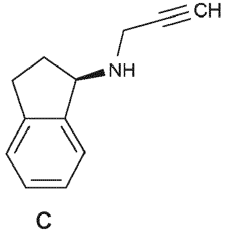

Neuroprotective and other pharmaceutical effects have also been described for inhibitors (Curr. Pharm. Des. 2010, 16: 2799-2817, Nature Reviews Neuroscience 2006, 295: 295-309; Br. J. Pharmacol. 2006, 147: 5287-5296, J. Alzheimers Dis. 2010, 21: 361-371, Prog. Neurobiol. 2010, 92: 330-344). MAO-B inhibitors are for example used to increase DOPA levels in CNS (Progr. Drug Res. 1992, 38: 171-297) and they have been used in clinical trials for the treatment of Alzheimer's disease (AD) based on the fact that an increased level of MAO-B is involved in astrocytes associated with Alzheimer plaques (Neuroscience 1994, 62: 15-30).

Fluorinated MAO inhibitors have been synthesised and biochemically evaluated (Kirk et al., Fluorine and Health, A. Tressaud and G. Haufe (editors), Elsevier 2008, pp. 662-699). ¹⁸F and ¹¹C labelled MAO inhibitors have been studied *in vivo* (Journal of the Neurological Science 2007, 255: 17-22; review: Methods 2002, 27: 263-277).

¹⁸F labelled deprenyl and deprenyl analogues (D) and (E) have also been reported (Int. J. Radiat. Appl. Instrument. Part A, Applied Radiation Isotopes, 1991, 42: 121; J. Med. Chem. 1990 33: 2015-2019 and Nucl. Med. Biol. 1990, 26: 111-116, respectively).

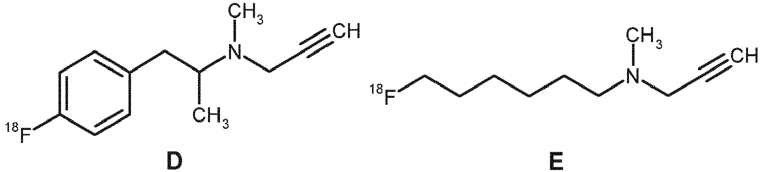

¹⁸F labelled rasagiline (compound **F**) has been reported in WO2009/052970A2.

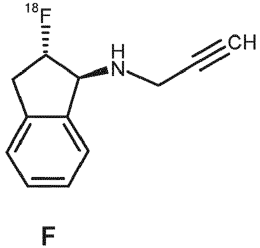

Amongst said ¹¹C labelled MAO inhibitors, [¹¹C]-L-Deprenyl-D2 (compound G), also referred to as DED ([¹¹C]-L-bis-deuterium-deprenyl), has been widely used by multiple groups to study CNS diseases with regard to their impact on MAO-B acitivity, such as epilepsy (Acta Neurol. Scand. 2001, 103: 360; Acta Neurol. Scand. 1998, 98: 224; Epilepsia 1995, 36: 712), amyotrophic lateral sclerosis (ALS, see J. Neurolog. Sci. 2007, 255: 17), and traumatic brain injury (Clin. Positron Imaging 1999, 2: 71).

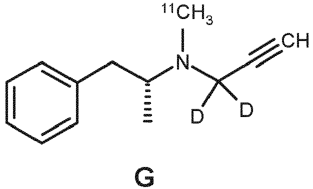

Moreover, a comparative multitracer study including compound G has been performed in patients suffering from Alzheimer's disease (AD) and healthy controls (Neurolmage 2006, 33: 588).

[¹¹C]-L-bis-deuterium-deprenyl (compound **G**) has been furthermore used in studies on the effect of smoking and age on MAO-B acitivity (Neurobiol. Aging 1997, 18: 431; Nucl. Med. Biol. 2005, 32: 521; Proc. Nat. Acad. Sci. USA 2003, 20: 11600; Life Sci. 1998, 63: 2, PL19; J. Addict. Disease 1998, 17: 23).

The non-deuterated analogue of compound G [¹¹C]-L-deprenyl binds very rapidly and irreversibly to MAO-B. As a result, the tracer may be trapped at a rate similar to or higher than its delivery by plasma, rendering PET images of regions with high MAO-B levels and/or low blood flow representing perfusion rather than MAO-B activity. The binding of compound **G** is slower due to a kinetic isotope effect and thus compound **G** allows for a more accurate assessment of MAO-B activity as its non-deuterated counterpart (see e.g. J. Nucl. Med. 1995, 36: 1255; J. Neurochem. 1988, 51: 1524).

However, in view of the MAO inhibitors disclosed in prior art, there is still need for novel compounds and methods for imaging diseases which go along with increased level of MAO enzyme, especially for novel imaging agents and methods which are easy to realize and which enable imaging certain levels of astrocyte activation and which give a superior signal to background level in order to increase the ability to detect also early changes, especially e.g. in cortical regions in subjects suffering from or likely to develop dementia or in AD patients.

### Problem to be solved and its solution

The aim of the present invention was to provide ¹⁸F labelled compounds selectively binding to MAO-B and featuring superior signal to background ratio as compared to the compounds disclosed in prior art. Another aim of the present invention was to provide suitable precursors for the preparation of such ¹⁸F labelled compounds.

This aim was achieved by the provision of the compounds of the present invention which showed excellent uptake of the inventive ¹⁸F labelled compounds in target regions.

Surprisingly it was observed that the signal intensity caused by deuterated [¹⁸F],(²H₂)rasagiline (compound **1**) bound to MAO-B in target regions is up to 3 times lower than in case of [¹⁸F]rasagiline (compound F) at steady state. The reduced trapping rate of deuterated [¹⁸F],(²H₂)rasagiline (compound **1**) compared to [¹⁸F]rasagiline (compound F) enables an improved quantification of MAO-B activity and avoids blood flow limitation of delivery of the tracer, since it is known that a high trapping rate is responsible for an underestimation of the MAO-B signal in regions with high MAO-B activity. The unexpected observation is that regions with high MAO-B expression had a more pronounced decrease in signal intensity when compared to the effect that was expected from data published for [¹¹C],(²H₂)deprenyl (compound **G**) and [¹¹C]deprenyl (compound **H**). The effect strongly exceeds effects reported for compounds from the prior art by up to two times and hence could not be expected by the person skilled in the art.

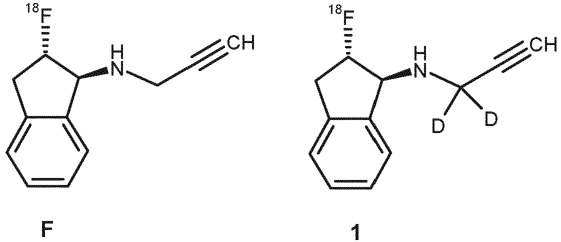

Surprisingly a reduction of the number of metabolites from eight (for [¹⁸F]rasagiline F) to two (for [¹⁸F], (²H₂)rasagiline, **1**) was observed. The data hint to an improved metabolism profile of [¹⁸F], (²H₂)rasagiline (**1**) as an advantage over the non-deuterated compound expecting to lead to less background signal in brain PET images.

### Summary of the Invention

This invention relates to [¹⁸F], (²H₂)rasagiline (compounds of formula **Ia**), the ¹⁹F-fluorinated analogues thereof (compound of formula **Ib**), to precursors (compounds of formula **Ic)** for preparing the ¹⁸F and ¹⁹F fluorinated compounds according to the invention, to the intermediates of formulae **Id** and **Ie**, and to the use of fluorinated compounds according to the invention for imaging diseases associated with altered expression of MAO-B or as reference standards, methods of preparing such compounds, compositions comprising such compounds, kits comprising such compounds or compositions and uses of such compounds, compositions or kits for diagnostic imaging by Positron Emission Tomography (PET). 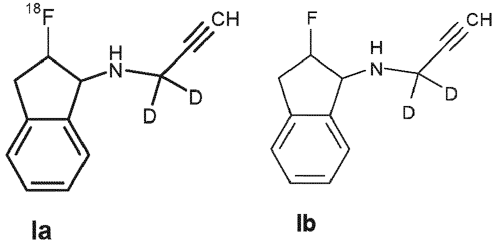 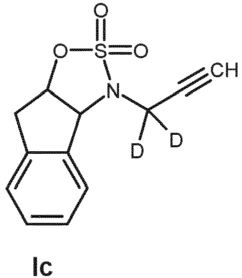 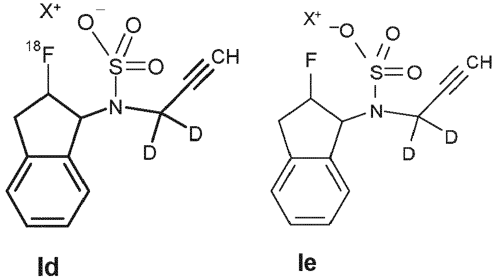 wherein X⁺ is selected from the group Li⁺, Na⁺, K⁺, Cs⁺, and/or Rb⁺.

### Detailed Description of the Invention

The present invention covers compounds of general formula I: 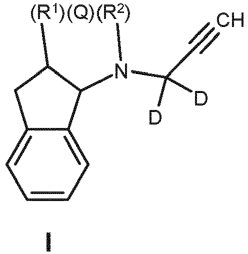 wherein
**(R¹)(Q)(R²)** is selected from the group consisting of (O)(bond)(S(O)₂),
([¹⁸F]fluoro)(blank)(S(O)₂(O⁻)(X⁺)),
(F)(blank)(S(O)₂(O⁻)(X⁺)),
([¹⁸F]fluoro)(blank)(H), and
(F)(blank)(H);
X⁺ is selected from the group consisting of a metal ion equivalent of lithium (Li), sodium (Na), potassium (K), caesium (Cs) and rubidium (Rb);
including all isomeric forms of said compound, including but not limited to enantiomers and diastereoisomers as well as mixtures of isomers, and any pharmaceutically acceptable salt or complex thereof.

The term "bond" as employed herein by itself or as part of another group means that the atoms or atom groups adjacent to the bond share at least one pair of electrons and thus are chemically bonded to each other.

In contrast to "bond", the term "blank" as employed herein by itself or as part of another group means that there is neither a chemical bond nor an atom or an atom group connecting the atoms adjacent to the "blank".

The terms "¹⁹F", "F-19", and "F" are used synonymously herein. A compound comprising ¹⁹F, F-19, or F is a compound, which exhibits a natural fluorine isotope distribution. A person skilled in the art knows that in nature fluorine exists as a Fluorine-19 isotope only. In other words, a compound comprising ¹⁹F, F-19, or F is a compound, where no efforts were made in order to enrich a certain fluorine isotope (e.g. ¹⁸F). Usually, the term "F" is used herein, however, sometimes the term "¹⁹F" or the term "F-19" is used in order to clearly discriminate such a fluorine compound from its ¹⁸F labeled analogue.

The terms "¹⁸F" and "F-18" are used synonymously herein. A compound comprising ¹⁸F, or F-18 is a compound enriched with the isotope Fluorine-18.

The terms "D" and "²H" are used synonymously herein. A compound comprising D, or ²H is a compound enriched with deuterium.

In accordance with a **first** aspect, the present invention is directed to [¹⁸F], (²H₂)rasagiline (compounds of formula **Ia**): 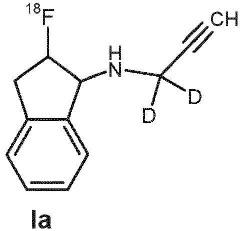 including all isomeric forms, including but not limited to enantiomers and diastereoisomers as well as mixtures of isomers, and any pharmaceutically acceptable salt thereof.

As will be shown below, the compounds of formula **Ia** [¹⁸F], (²H₂)rasagiline) selectively bind to MAO-B, and can be used as a tracer for PET imaging, thereby featuring superior signal to background ratio as compared to the compounds of prior art.

In accordance with a **second** aspect, the present invention is directed to compounds of formula **Ib:** 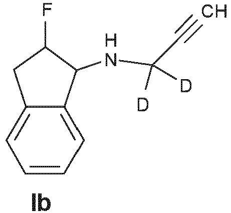 including all isomeric forms, including but not limited to enantiomers and diastereoisomers as well as mixtures of isomers, and any pharmaceutically acceptable salt thereof.

The ¹⁹F analogues **Ib** of the PET tracers **Ia** can be used as a reference standard.

In accordance with a third aspect, the present invention is directed to compounds of formula **Ic:** 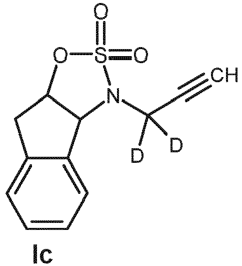 including all isomeric forms, including but not limited to enantiomers and diastereoisomers as well as mixtures of isomers, and any pharmaceutically acceptable salt thereof.

The compounds of formula **Ic** can be used as precursors for the preparation of the fluorinated compounds of formulae **Ia** and **Ib**.

In accordance with a **fourth** aspect, the present invention is directed to compounds of formulae **Id** and **Ie:** 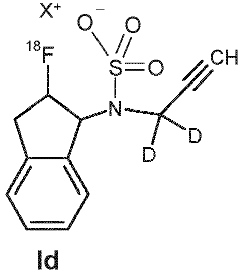 wherein X⁺ is selected from the group Li⁺, Na⁺, K⁺, Cs⁺, and/or Rb⁺; in a preferred embodiment of the present invention X⁺ is K⁺;
including all isomeric forms, including but not limited to enantiomers and diastereoisomers as well as mixtures of isomers, and any pharmaceutically acceptable salt thereof. 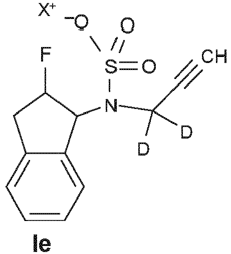 wherein X⁺ is selected from the group Li⁺, Na⁺, K⁺, Cs⁺, and/or Rb⁺; in a preferred embodiment of the present invention X⁺ is Cs⁺;
including all isomeric forms, including but not limited to enantiomers and diastereoisomers as well as mixtures of isomers, and any pharmaceutically acceptable salt thereof.

The compounds of formulae **Id** and **Ie** are intermediates arising during preparation of compounds of formulae **Ia** and **Ib** and/or precursors of compounds of formulae **Ia** and **Ib**.

From the purposes of the respective compounds disclosed above, it is apparent that the compounds of formulae **Ia, Ib, Ic, Id,** and **Ie** are linked by the technical problem to be solved in view of prior art, i.e. the provision of improved PET tracers for MAO-B imaging, as well as by the inventive solution, and therefore form a single general inventive concept.

If chiral centres or other forms of isomeric centres are not otherwise defined in a compound according to the present invention, all forms of such stereoisomers, including enantiomers and diastereoisomers, are intended to be covered herein. Compounds containing chiral centres may be used as racemic mixture or as an enantiomerically enriched mixture or as a diastereomeric mixture or as a diastereomerically enriched mixture, or these isomeric mixtures may be separated using well-known techniques, and an individual stereoisomer maybe used alone.

Pharmaceutically acceptable salts of the compounds according to the invention include salts of mineral acids, carboxylic acids and sulfonic acids, for example salts of hydrochloric acid, hydrobromic acid, sulfuric acid, phosphoric acid, methanesulfonic acid, ethanesulfonic acid, toluenesulfonic acid, benzenesulfonic acid, naphthalene disulfonic acid, acetic acid, trifluoroacetic acid, propionic acid, lactic acid, tartaric acid, malic acid, citric acid, fumaric acid, maleic acid and benzoic acid.

Pharmaceutically acceptable salts of the compounds according to the invention also include salts of customary bases, such as, by way of example and by way of preference, alkali metal salts (for example sodium salts and potassium salts), alkaline earth metal salts (for example calcium salts and magnesium salts) and ammonium salts, derived from ammonia or organic amines having 1 to 16 carbon atoms, such as, by way of example and by way of preference, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

As used herein, the term "carrier" refers to microcrystalline cellulose, lactose, mannitol.

As used herein, the term "solvents" refers to liquid polyethylene glycols, ethanol, corn oil, cottonseed oil, glycerol, isopropanol, mineral oil, oleic acid, peanut oil, purified water, water for injection, sterile water for injection and sterile water for irrigation.

As used herein, the term "stabilizers" refers to antioxidants, such as, ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite.

"MAO-B activity" refers to an enzymatic activity that catalyzes the deamination of monoamines, i.e. neurotransmitters and bioactive amines.

Preferred compounds of formula I are: 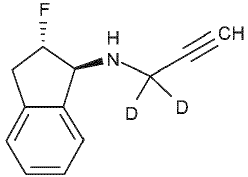 (1*S*,2*S*)-2-fluoro-*N*-[(1,1-²H₂)prop-2-yn-1-yl]indan-1-amine 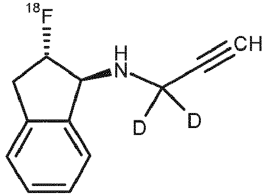 (1*S*,2*S*)-2-[¹⁸F]fluoro-*N*-[(1,1-²H₂)prop-2-yn-1-yl]indan-1-amine 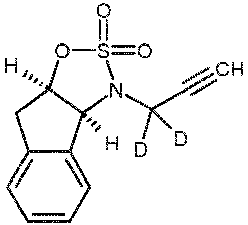 (3a*S*,8a*R*)-3-[(1,1-²H₂)prop-2-yn-1-yl]-3,3a,8,8a-tetrahydroindeno[1,2-d][1,2,3]oxathiazole 2,2-dioxide 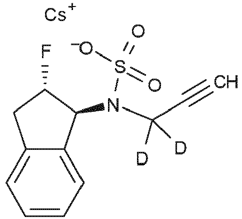 caesium *N*-[(1*S*,2*S*)-2-fluoroindan-1-yl]-*N*-[(1,1-²H₂)prop-2-yn-1-yl]sulfamate 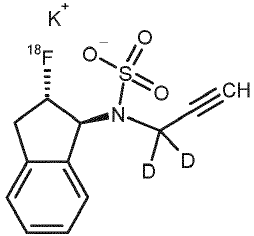 potassium *N*-[(1*S*,2*S*)-2-[¹⁸F]fluoroindan-1-yl]-*N*-[(1,1-²H₂)prop-2-yn-1-yl]sulfamate

In a **fifth** aspect, the invention is directed to a composition comprising a compound of formula I 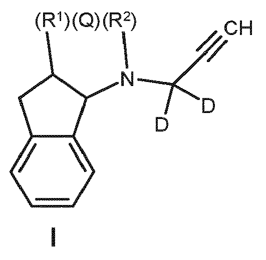 wherein
**(R¹)(Q)(R²)** is selected from the group consisting of
(O)(bond)(S(O)₂),
([¹⁸F]fluoro)(blank)(S(O)₂(O-)(X⁺)),
(F)(blank)(S(O)₂(O-)(X⁺)),
([¹⁸F]fluoro)(blank)(H), and
(F)(blank)(H);
wherein X⁺ is selected from the group Li⁺, Na⁺, K⁺, Cs⁺ and/or Rb⁺; or a pharmaceutically acceptable salt of an inorganic or organic acid or a hydrate thereof.

Preferably, the composition comprises a physiologically acceptable carrier, diluent, adjuvant or excipient.

In a first embodiment, the invention is directed to a composition comprising a compound of formula **Ia** and one or more pharmaceutically suitable adjuvants. These adjuvants include, inter alia, carriers, solvents, and/or stabilizers.

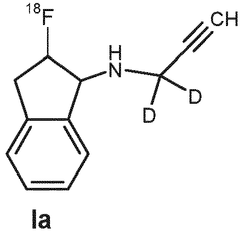

The person skilled in the art is familiar with adjuvants which are suitable for the desired pharmaceutical formulations, preparations or compositions on account of his/her expert knowledge.

The administration of the compounds, pharmaceutical compositions or combinations according to the invention is performed in any of the generally accepted modes of administration available in the art. Intravenous deliveries are preferred.

Preferably, the compositions according to the invention are administered such that the dose of the active compound for imaging is in the range of 37 MBq (1 mCi) to 740 MBq (20 mCi). In particular, a dose in the range from 100 MBq to 400 MBq will be used.

Preferably, the composition comprises (1*S*,2*S*)-2-[¹⁸F]fluoro-*N*-[(1,1-²H₂)prop-2-yn-1-yl]indan-1-amine:

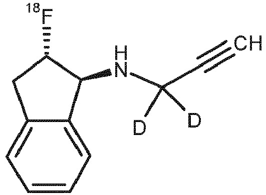

In a second embodiment, the invention is directed to a composition comprising a compound of formula **Ib.**

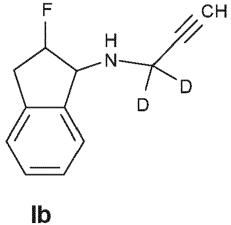

Such composition can be used for analytical purposes. Preferably, the composition comprises (1*S*,2*S*)-2-fluoro-*N*-[(1,1-²H₂)prop-2-yn-1-yl]indan-1-amine:

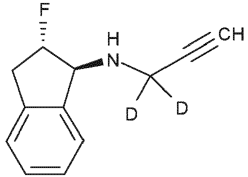

In a third embodiment, the invention is directed to a composition comprising a compound of formula **Ic.**

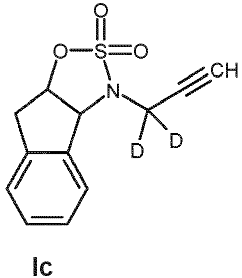

Such composition can be used for manufacturing of compounds of formula **Ia** and/or **Ib** and for analytical purposes.

Preferably, the composition comprises (3a*S*,8a*R*)-3-[(1,1-²H₂)prop-2-yn-1-yl]-3,3a,8,8a-tetrahydroindeno[1,2-*d*][1,2,3]oxathiazole 2,2-dioxide:

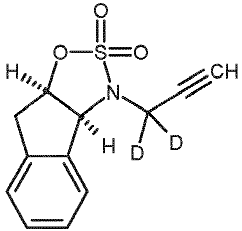

In a **sixth** aspect, the invention is directed to methods for obtaining compounds of formula **I.**

Six methods have been identified for obtaining compounds of formula **I**:

### Synthesis of compounds of formula Ia from compounds of formula Id

The method for obtaining compounds of formula **Ia** comprises the step
- reacting a compound of formula **Id** with acid.

The preferred features and embodiments disclosed for compounds of general formula **Ia** are herein incorporated.

### Synthesis of compounds of formula Ia from compounds of formula Ic

The method for obtaining compounds of formula **Ia** comprises the steps
- reacting a compound of formula **Ic** with an ¹⁸F-fluorination agent,
- deprotecting the obtained compound **Id** for obtaining compound of formula **Ia**, and
- optionally, converting obtained compound into a suitable salt of inorganic or organic bases, hydrates, and/or solvates thereof.

The preferred features and embodiments disclosed for compounds of general formula **Ia** are herein incorporated.

### Synthesis of compounds of formula Id from compounds of formula Ic

The method for obtaining compounds of formula **Ia** comprises the step
- reacting a compound of formula **Ic** with a ¹⁸F-fluorination agent.

The preferred features and embodiments disclosed for compounds of general formula **Id** are herein incorporated.

### Synthesis of compounds of formula Ib from compounds of formula Ie

The method for obtaining compounds of formula **Ib** comprises the step
- reacting a compound of formula **Ie** with acid.

The preferred features and embodiments disclosed for compounds of general formula **Ib** are herein incorporated.

### Synthesis of compounds of formula Ib from compounds of formula Ic

The method for obtaining compounds of formula **Ib** comprises the steps
- reacting a compound of formula **Ic** with a ¹⁹F-fluorination agent,
- deprotecting the obtained compound for obtaining compound of formula **Ib,** and
- optionally converting obtained compound into a suitable salt of inorganic or organic bases, hydrates, complexes, and/or solvates thereof.

The preferred features and embodiments disclosed for compounds of general formula **Ib** are herein incorporated.

### Synthesis of compounds of formula Ie from compounds of formula Ic

The method for obtaining compounds of formula **Ie** comprises the step
- reacting a compound of formula **Ic** with a ¹⁹F-fluorination agent.

The preferred features and embodiments disclosed for compounds of general formula **Ie** are herein incorporated.

The ¹⁸F-fluorination agent can be chelated complexes known to those skilled in the art, e.g. 4,7,13,16,21,24-Hexaoxa-1,10-diazabicyclo[8.8.8]-hexacosane K¹⁸F (crown ether salt Kryptofix K¹⁸F), 18-crown-6 ether salt K¹⁸F, K¹⁸F, H¹⁸F, KH¹⁸F₂, Rb¹⁸F, Cs¹⁸F, Na¹⁸F, or tetraalkylammonium salts of ¹⁸F known to those skilled in the art, e.g.[¹⁸F] tetrabutylammonium fluoride, or tetraalkylphosphonium salts of ¹⁸F known to those skilled in the art, e.g.[¹⁸F] tetrabutylphosphonium fluoride. Most preferably, the ¹⁸F-fluorination agent is Cs¹⁸F, K¹⁸F, H¹⁸F, or KH¹⁸F₂.

The ¹⁹F-fluorination agent is a reagent suitable for substituting the -OS(=O)₂NR³R⁴-moiety in the compound of formula **Ic** by a ¹⁹F atom. Such reagents are exemplified by but not limited to inorganic salts and/or adducts of hydrofluoric acid, e.g. sodium fluoride, potassium fluoride, potassium hydrogen difluoride, or caesium fluoride as such or in combination with chelating reagents, e.g. aminopolyether 2.2.2 (K2.2.2) ; organic salts and/or adducts of hydrofluoric acid such as tetra n-butylammonium fluoride (TBAF) or triethylamine tris-hydrofluoride; hypervalent fluorosilicates, e.g. tetrabutylammonium triphenyldifluorosilicate; sulfur fluorides, e.g. (diethylamino)sulfur trifluoride (DAST); sulfonyl fluorides, e.g. 1,1,2,2,3,3,4,4,4-nonafluorobutane-1-sulfonyl fluoride; also electrophilic fluorination reagents are suitable to introduce ¹⁹F into organic molecules, such as *N*-fluoropyridinium salts, e.g. N-fluoropyridinium triflate; N-fluorosulfonimides, e.g. *N*-fluorobenzenesulfonimide; aliphatic N-fluoroamines derivatives, e.g. 1-chloromethyl-4-fluoro-1,4-diazoniabicyclo[2.2.2]octane bis(tetrafluoroborate) (Selectfluor®). As known to the person skilled in the art, said reagents may be used alone or in combination, e.g. 1,1,2,2,3,3,4,4,4-nonafluorobutane-1-sulfonyl fluoride in combination with triethylamine tris-hydrofluoride. For further methods, see publications in reach of the person skilled in the art, e.g. Ritter T. et al. Current Opinion in Drug Discovery & Development 2008, 11(6), 803-819, Kirk, K.L. Organic Process Research & Development 2008, 12, 305-321, and references cited therein.

The reagents, solvents and conditions which can be used for this fluorination are common and well-known to the skilled person in the field. See, *e.g*., S. L. Pimlott, A. Sutherland, Chem. Soc. Rev. 2011, in press, DOI:10.1039/b922628c; Z. Li, P. S. Conti, Adv. Drug Deliv. Rev. 2010, 62, 1031; P. W. Miller, N. J. Long, R. Vilar, A. D. Gee, Angew. Chem. Int. Ed. 2008, 47, 8998; L. Cai, S. Lu, V. W. Pike, Eur. J. Org. Chem. 2008, 2853; G. Angelini, M. Speranza, A. P. Wolf, C.-Y. Shiue, J. Fluorine Chem., 1985, 27, 177-191. Preferably, the solvents used in the present method are DMF, DMSO, acetonitrile, DMA, or mixture thereof, preferably the solvent is DMSO.

Embodiments and preferred features can be combined together and are within the scope of the invention.

In a **seventh** aspect, the invention also provides for an ¹⁸F-labelled compound of formulae **Ia** or a composition containing such compound for use as a diagnostic agent or imaging agent.

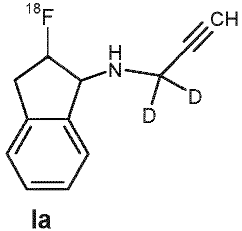

Preferably, the compound or composition is used as an imaging tracer or radiopharmaceutical agent for imaging diseases associated with altered expression of MAO-B.

Preferably, altered expression of MAO-B refers to elevated expression of MAO-B.

In other words, the invention is directed to the use of a compound of formula **Ia** for the manufacture of a radiopharmaceutical imaging tracer/agent for imaging diseases associated with elevated expression of MAO-B.

The compounds of general formula **Ia** are herein defined as above and encompass all embodiments and preferred features.

The radiopharmaceutical imaging tracer/agent is a Positron Emission Tomography (PET) suitable radiopharmaceutical imaging tracer/agent.

The invention is directed to a compound of general formula **Ia** for use in the imaging of diseases associated with elevated expression of MAO-B.

The disclosure is also directed to a method for imaging or diagnosing of diseases associated with elevated expression of MAO-B comprising the steps:
- administering to a mammal an effective amount of a composition comprising a compound of formula **Ia**,
- obtaining images of the mammal and
- assessing images.

Preferably, mammal is human.

Preferably, the compound of general formula **Ia** is (1*S*,2*S*)-2-[¹⁸F]fluoro-*N*-[(1,1-²H₂)prop-2-yn-1-yl]indan-1-amine.

Preferably, said diseases relate to inflamed CNS tissue; more preferably, said diseases relate to AD, multiple sclerosis and stroke; more preferably, said diseases relate to AD and multiple sclerosis; even more preferably, said diseases relate to AD.

In an **eighth** aspect, the disclosure , is directed to the use of a compound of formula **Ic** as a precursor for the manufacture of a radiopharmaceutical imaging tracer/agent of formula **Ia**.

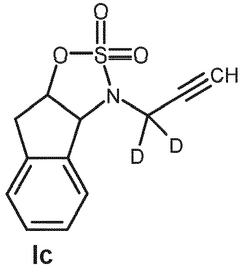

The compounds of general formula **Ic** are herein defined as above and encompass all embodiments and preferred features.

In a **ninth** aspect, the invention is directed to the use of compounds of general formula **I**, **Ia** or **Ib** for conducting biological assays and chromatographic identification. Compounds of general formula **Ib** are useful as references and/or measurement agents. The compounds of general formula **I**, **Ia** and **Ib** are herein defined as above and encompass all embodiments and preferred features.

In a **tenth** aspect, the disclosure , is directed to a method for inhibiting MAO-B activity by contacting inventive compounds of formula **I** with proteins exhibiting MAO-B activity *in-vitro* or *in-vivo*. Additionally, compounds of formula **Ia** and formula **Ib** can be coupled to a detectable label e.g. to fluorescent dyes. The compounds of general formula **I**, **Ia** and **Ib** are herein defined as above and encompass all embodiments and preferred features.

In an **eleventh** aspect, the present invention is directed to a kit comprising at least one sealed vial containing a predetermined quantity of a compound of formula **I**, **Ia, Ib, Ic, Id,** and/or **Ie**. The compounds of general formula **I**, **Ia, Ib, Ic, Id,** and **Ie** are herein defined as above and encompass all embodiments and preferred features.

Optionally, the kit comprises a pharmaceutically acceptable carrier, diluent, excipient or adjuvant.

Preferably, the kit comprises a predefined quantity of compound of formula **I**, **Ib** or **Id,** and one or more solid-phase extraction cartridges/columns for the purification of compound of formula I or formula **Ia**.

Preferably, the kit comprises physiologically acceptable vehicle or carrier and optional adjuvants and preservatives, reagents suitable to perform the herein disclosed reactions and/or methods to generate the [¹⁸F] labeling reagents.

Furthermore, the kit may contain instructions for its use.

### General synthesis of compounds of the invention

Compounds of formula I can be synthesized starting e.g. from *cis*-1-aminoindan-2-ol (2) (compare scheme 1).

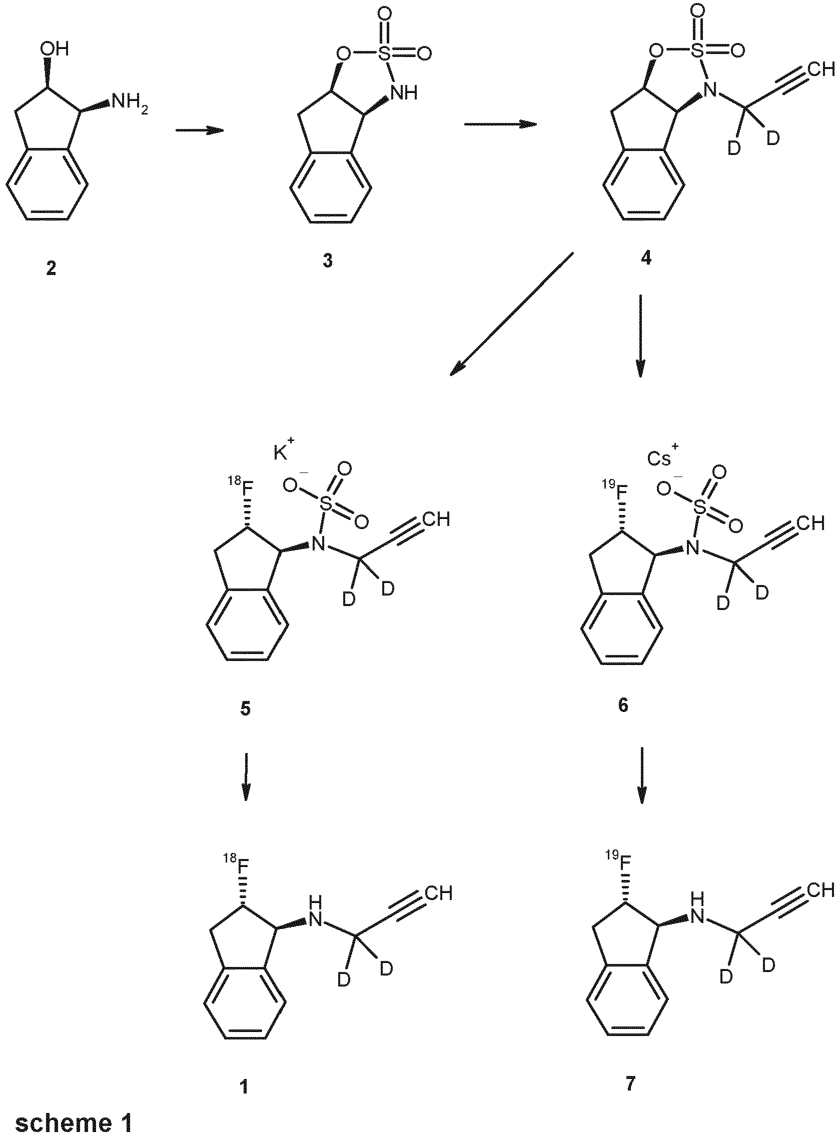

The amino alcohol **2** is converted to the cyclic sulphamidate **3** using sulfuryl chloride. Tricyclic sulphamidate **3** is alkylated by (1,1-²H₂)prop-2-yn-1-yl 4-methylbenzenesulfonate which can be prepared from (1,1-²H₂)prop-2-yn-1-ol (Nucl. Med. Biol. 2001, 28 (7), 779 - 785) using tosyl anhydride. It is obvious to someone skilled in the art that the resulting sulphamidate **4** could also be obtained from **3** and (1,1-²H₂)prop-2-yn-1-ol by Mitsonobu reaction conditions. Resulting sulphamidate **4** is converted to ¹⁸F and ¹⁹F fluorinated bicyclic compounds **5** and **6**, respectively, using potassium fluoride and caesium fluoride, respectively, or other ¹⁸F and ¹⁹F fluorinating agents. Aqueous hydrogenchloride is added subsequently to the reaction mixture leading to fluorides **1** and **7**, respectively. It is obvious to someone skilled in the art that also other appropriate acids are possible for this reaction, e.g. organic acids and mineral acids, e.g. sulphuric acid.

### Description of the drawings

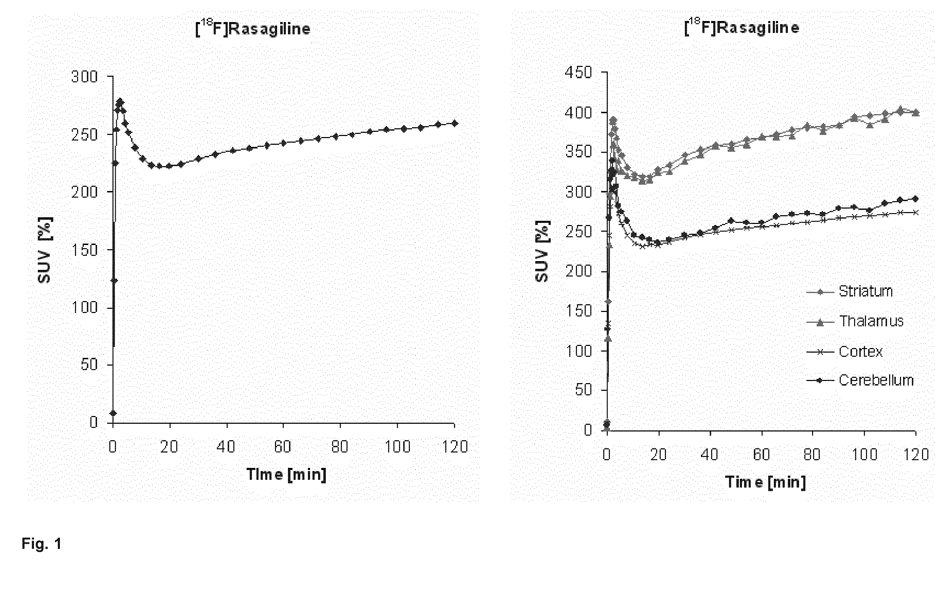

**Fig. 1****:** Cynomolgus monkeys were used to monitor [¹⁸F]rasagiline (compound **F**) uptake and distribution in the brain *in vivo* using PET. **Left panel:** [¹⁸F]rasagiline (compound **F**) is taken up in the monkey brain with ca. 280 %SUV, slightly eliminated within 15 minutes to reach ca. 225 %SUV and thereafter, increases slightly over time (by about 35 %SUV until the end of the observation time). **Right panel:** This panel demonstrates the time activity curves (TACs) within specific regions of interest, i.e. Striatum, Thalamus, Cortex and Cerebellum. Note that the TACs for the striatum and thalamus, both regions with high MAO-B activity, levelled at comparable values as did the cortical and cerebellar TACs, both regions with low levels of MAO-B activity, but at a lower level.

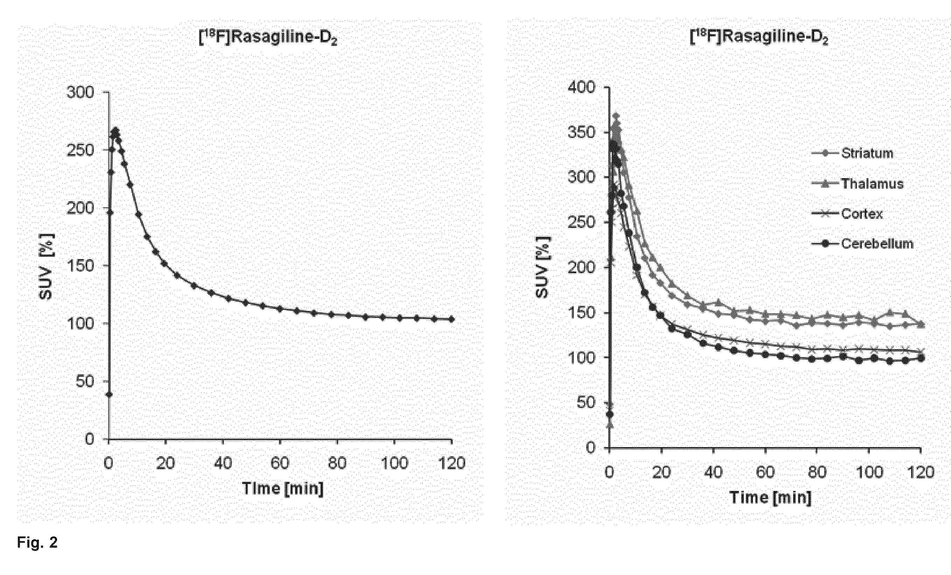

**Fig. 2****:** Cynomolgus monkeys were used to monitor [¹⁸F], (²H₂)rasagiline (**1**) uptake and distribution in the brain *in vivo* using PET. **Left panel:** [¹⁸F], (²H₂)rasagiline (**1**) is taken up in the monkey brain with an initial amount comparable to [¹⁸F]rasagiline (compound **F**). However, after the initial uptake the radioactivity is eliminated in a manner resembling that of a reversible ligand. **Right panel:** This panel demonstrates the TACs within specific regions of interest, i.e. Striatum, Thalamus, Cortex and Cerebellum. Note that the TACs for the striatum and thalamus (regions with high MAO-B activity) levelled at comparable values as did the cortical and cerebellar (regions with low MAO-B activity) TACs, but at a lower level. Also for the regions of interest investigated the elimination behaviour of the radioactivity was comparable to that of a reversible ligand.

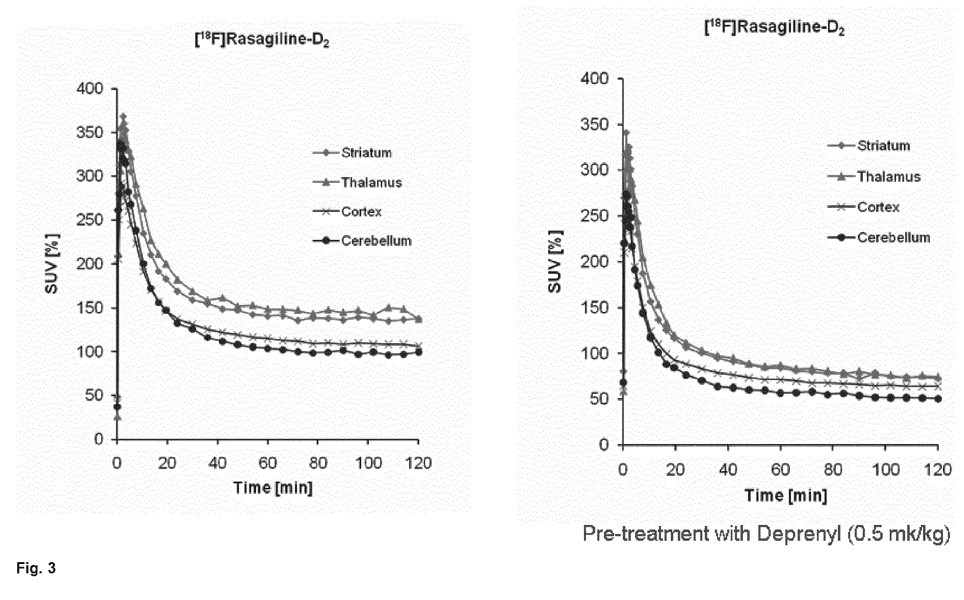

**Fig. 3**: The brain TACs for [¹⁸F], (²H₂)rasagiline (**1**) within the striatum, thalamus, cortex and cerebellum are shown in the left panel and compared to the brain TACs for the same regions as have been measured after pre-treatment with 0.5 mg/ kg deprenyl before injection of a comparable amount of [¹⁸F], (²H₂)rasagiline (**1**). Note the overall decrease in %SUV.

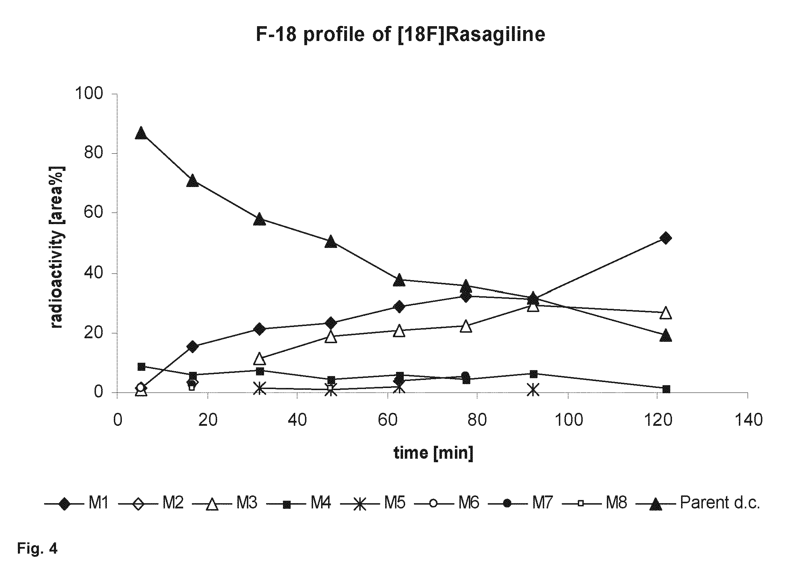

**Fig. 4**: The radioactivity profile of the [¹⁸F]rasagiline (compound **F**) as well as the profile of metabolites labelled with ¹⁸F is demonstrated. Eight metabolites were detected. The data were plotted as area% for each time point investigated calculated from the radio HPLC chromatogram. d.c.= decay corrected

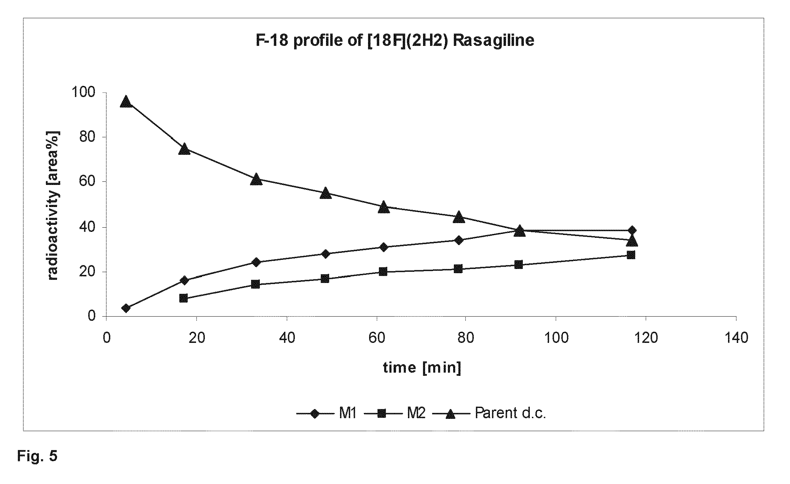

**Fig. 5****:** The radioactivity profile of [¹⁸F], (²H₂)rasagiline (**1**) as well as the profile of metabolites labelled with ¹⁸F is demonstrated. Surprisingly, only two metabolites were detected. The data were plotted as area% for each time point investigated calculated from the radio HPLC chromatogram. d.c.= decay corrected

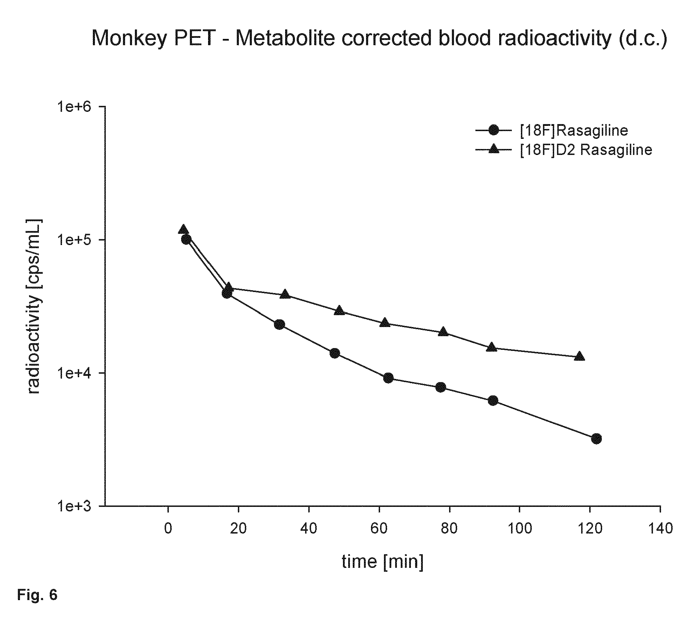

**Fig. 6****:** The blood radioactivity time curves that were corrected for the respective metabolite fractions at each time point are shown for [¹⁸F]rasagiline (compound **F**) and [¹⁸F], (²H₂)rasagiline (**1**), respectively. Note the slower elimination for the di-deuterated compound (**1**). d.c.= decay corrected

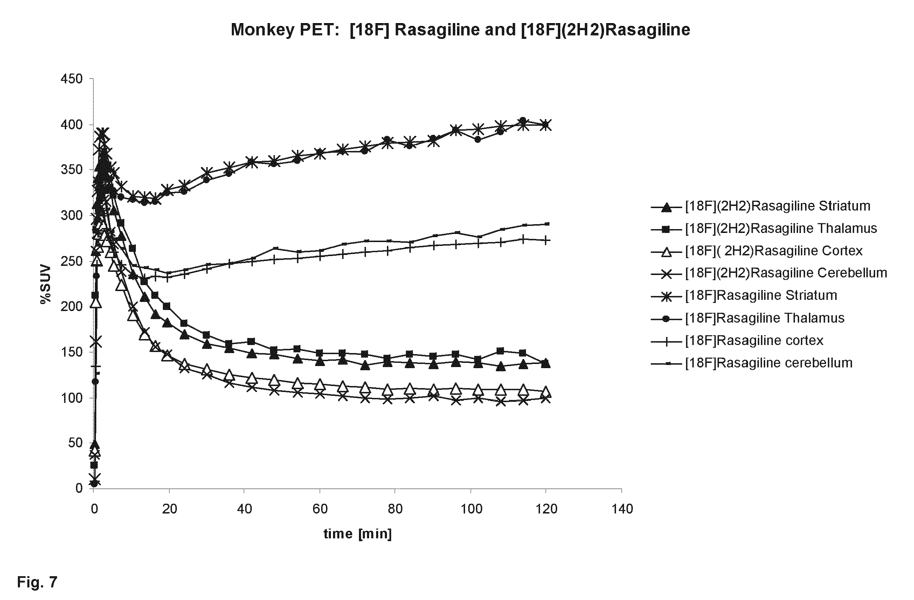

**Fig. 7****:** PET time activity curves (TAC) expressed as percent SUV of [¹⁸F], (²H₂)rasagiline (**1**) over a time of 120 min and compared to the respective TACs of non-deuterated [¹⁸F]rasagiline (compound **F**). Surprisingly, the decrease of the signal of [¹⁸F], (²H₂)rasagiline (**1**), as expressed as % standard uptake value (SUV) in TACs compared to the signal of the non-deuterated [¹⁸F]rasagiline (compound **F**) was 2.4 to 3 times between 30 and 120 min in the investigated brain regions of cynomolgus monkeys. This was not expected since the decrease in signal due to the deuteration effect known from [¹¹C]deprenyl (compound **H**) versus [¹¹C],(²H₂)deprenyl (compound **G**) is only approximately 1.2 - 2.0 times observed in baboon and human brain regions comparable to those investigated by us, e.g. striatum, thalamus, cortex (Fowler et al. J. Neurochem. 1988, 51: 1524-1534; J. Nucl. Med. 1995, 36: 1255-1262; Mol. Imaging Biol. 2005, 7: 377-387).
   Thus, the brain trapping also in target regions was less pronounced and leads to an advantage over [¹¹C],(²H₂)deprenyl (compound **G**) regarding background signal.

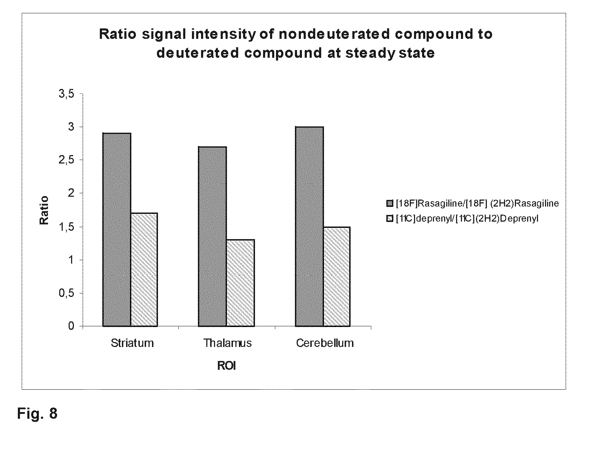

**Fig. 8****:** Graphic visualization of the effect of deuteration of [¹⁸F]rasagiline for signal retention in target (thalamus and striatum) and non-target (cerebellum) areas as compared to the effect of deuteration of [¹¹C]deprenyl reported in the literature.

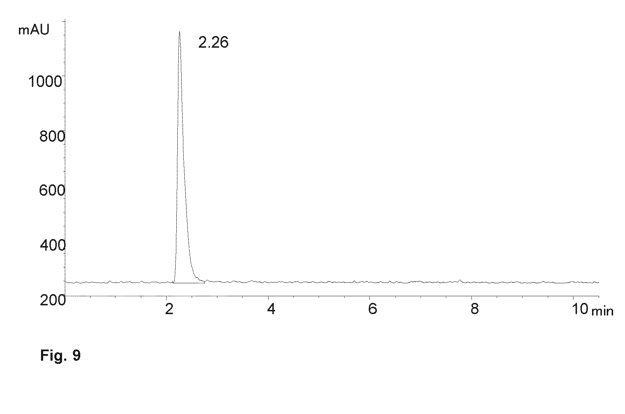

**Fig. 9****:** Analytical HPLC chromatogram of deuterated [¹⁸F],(²H₂)rasagiline **example 3** (gamma detection)

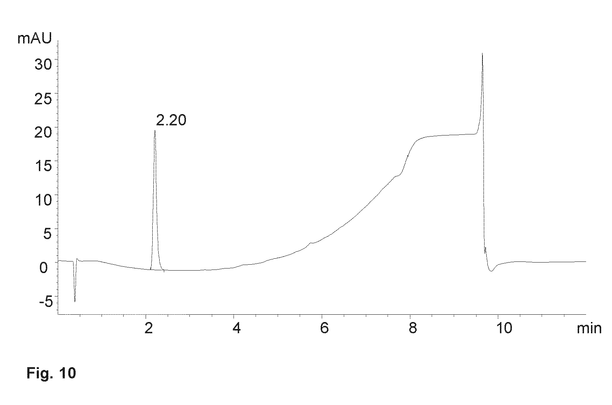

**Fig. 10****:** Analytical HPLC chromatogram of deuterated [¹⁸F],(²H₂)rasagiline **example 2** (UV detection)

**Table 1:** Data which are graphically depicted in Fig. 4

**Table 2:** Data which are graphically depicted in Fig. 5

### Experimental section

**General:** All solvents and chemicals were obtained from commercial sources and used without further purification if not stated otherwise. The following table lists the abbreviations used in this paragraph and in the Examples section as far as they are not explained within the text body.

**Abbreviations**

| | |
|---|---|
| CI | chemical ionisation |
| d | doublet (in NMR) |
| dd | doublet of doublet |
| DMF | *N*,*N*-dimethylformamide |
| DMSO | dimethylsulfoxide |
| ESI | electrospray ionisation |
| EtOAc | ethyl acetate |
| h | hour |
| K₂CO₃ | potassium carbonate |
| K_{2.2.2} | 4, 7, 13, 16, 21, 24-hexaoxa-1,10-diazabicyclo[8.8.8]-hexacosane |
| MeCN | acetonitrile |
| MS | mass spectrometry |
| m | multiplet |
| min | minute |
| NMR | nuclear magnetic resonance spectroscopy : chemical shifts (δ) are given in ppm. |
| r.t. | room temperature |
| s | singlet |
| sec | second |
| t | triplet |
| td | triplet of doublet |

NMR peak forms are stated as they appear in the spectra, possible higher order effects have not been considered.

The compounds and intermediates produced according to the methods of the invention may require purification. Purification of organic compounds is well known to the person skilled in the art and there may be several ways of purifying the same compound. In some cases, no purification may be necessary. In certain cases, the compounds may be purified by crystallization. In some cases, impurities may be removed by trituration using a suitable solvent. In some cases, the compounds may be purified by chromatography, particularly flash column chromatography, using for example prepacked silica gel cartridges, e.g. from Separtis such as Isolute® Flash silica gel or Isolute® Flash NH₂ silica gel in combination with e.g. a FlashMaster II autopurifier (Argonaut/Biotage) and eluents such as gradients of hexane/EtOAc or dichloromethane/ethanol. In some cases, the compounds may be purified by preparative HPLC using for example a Waters auto purifier equipped with a diode array detector and/or on-line electrospray ionization mass spectrometer in combination with a suitable prepacked reverse phase column and eluents such as gradients of water and acetonitrile which may contain additives such as trifluoroacetic acid or aqueous ammonia. In some cases, purification methods as described above can provide those compounds of the present invention which possess a sufficiently basic functionality in the form of a salt, such as, in the case of a compound of the present invention which is sufficiently basic, a trifluoroacetate or formate salt for example. A salt of this type may be transformed into its free base form, respectively, by various methods known to the person skilled in the art.

### Preparation of Intermediates

### Intermediate 1A: (1,1-²H₂)prop-2-yn-1-yl 4-methylbenzenesulfonate

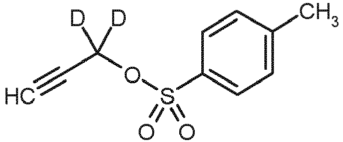

To a solution of (1,1-²H₂)prop-2-yn-1-ol (3.40 g, prepared according to Fowler et al., Nucl. Med. Biol. 2001, 28 (7): 779 - 785, separation from residual ethanol was accomplished by fractional distillation) in dichloromethane (250 mL) pyridine (7 mL) was added, and the resulting mixture was cooled to 0°C. Tosyl anhydride (21.0 g, 1.1 eq) was added to the mixture and the mixture was allowed to stir for 30 min, the cooling bath was removed and stirring was continued for 1.5 h. The mixture was concentrated in vacuo and the residue was purified by column chromatography on silica gel (EtOAc in hexane 2.5% → 25 %) to give the target compound in approx. 90 % puritiy (9.39 g, 68 % yield).
¹H NMR (300 MHz, CDCl₃) δ ppm 2.47 (m app s, 4 H), 7.36 (d, 2 H), 7.83 (d, 2 H).
MS (ESI): [M + H]⁺ = 213.

### Intermediate 1B: (3aS,8aR)-3,3a,8,8a-tetrahydroindeno[1,2-d][1,2,3]oxathiazole 2,2-dioxide

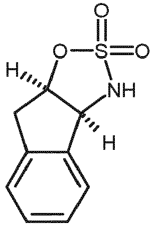

To a solution of commercially available (-)-(1*S*,2*R*)-*cis*-1-aminoindan-2-ol (12.8 g, 86 mmol) in dichloromethane (200 mL) triethylamine (29.9 mL, 214 mmol) was added, and the resulting mixture was cooled to -65°C by means of acetone/dry ice. A solution of sulfuryl chloride (8.27 mL, 103 mmol) in dichloromethane (250 mL) was added slowly (approx. 1 drop / sec) whilst maintaining temperature at -65°C. Upon complete addition, the cooling bath was removed and the mixture was allowed to warm up to room temperature. Stirring at room temperature was continued for 24 h. The mixture was washed twice with 2 N hydrochloric acid (200 mL each); the combined aqueous layers were extracted with dichloromethane (200 mL). The combined organic layers were dried over sodium sulfate and evaporated. The crude residue was purified by column chromatography on silica gel (EtOAc in hexane 0 % → 100 %), followed by trituration in hexane, gave an approx. 90 % pure product (5.6 g, 28 % yield). Repeated chromatography as described yielded a highly pure product.
¹H NMR (300 MHz, d₆-DMSO) δ ppm 3.19 (d, 1 H), 3.35 (dd, 1 H), 5.29 (t, 1 H), 5.52 (td, 1H), 7.14 - 7.44 (m, 4H), 8.32 (d, 1H).
MS (ESI): [M - H]⁻ = 210.

### Preparation of compounds according to the invention

### Example 1: (3aS,8aR)-3-[(1,1-²H₂)prop-2-yn-1-yl]-3,3a,8,8a-tetrahydroindeno[1,2-d][1,2,3]oxathiazole 2,2-dioxide

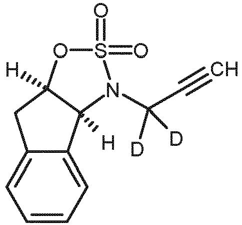

To a solution of Intermediate 1B (800 mg, 3,79 mmol) in DMF (30 mL) Intermediate 1A (1.29 g, 6.06 mmol) was added, followed by potassium carbonate (325 mesh, 2.09 g, 15.1 mmol), and sodium iodide (63 mg, 0.38 mmol). The resulting mixture was stirred at room temperature for 48 h, and then partitioned between ethyl acetate and water. The aqueous layer was extracted twice with ethyl acetate, and the combined organic layers were washed with brine, dried over sodium sulfate and evaporated. The residue was subjected to column chromatography on silica gel (EtOAc in hexane 0 % → 100 %) to give the desired product, partly in crystalline form, in a combined yield of 77 % (795 mg).
¹H NMR (400 MHz, CDCl₃) δ ppm 2.53 (s, 1 H), 3.48 (d, 2 H), 5.27 (d, 2 H), 5.53 - 5.60 (m, 1 H), 7.28 - 7.41 (m, 3 H), 7.49 (d, 1 H).
MS (ESI): [M + H]⁺ = 252.

### Example 2: (1S,2S)-2-fluoro-N-[(1,1-²H₂)prop-2-yn-1-yl]indan-1-amine

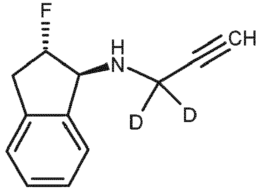

To a solution of Example 1 (430 mg, 1.71 mmol) in a mixture of DMF (2.5 mL) and *tert-*butanol (25 mL) caesium fluoride (1.17 g, 7.70 mmol) was added, and the resulting mixture was stirred at room temperature for 60 h. All volatiles were then removed *in vacuo*, followed by addition of 4 N hydrochloric acid (15 mL) and ethanol (15 mL) to the residue. The resulting mixture was stirred at 80 °C for 1.5 h, allowed to cool to room temperature, and then partitioned between ethyl acetate and saturated aqueous sodium carbonate. The aqueous layer was extracted with ethyl acetate and the combined organic layers were washed with brine, dried over sodium sulfate, and carefully evaporated (product is somewhat volatile). The residue was purified by column chromatography on silica gel (EtOAc in hexane 0% → 40%) to give the desired product (241 mg, 74 % yield). Highly pure material (130 mg) was obtained by short-path distillation of an aliquot (195 mg) using a Kugelrohr distillation apparatus (136 °C / 0.3 mbar) to give a colourless, crystalline solid upon cooling to room temperature.
A sample of the material was submitted to X-ray crystallography analysis confirming the assigned absolute stereochemistry.
¹H-NMR (400MHz, CHLOROFORM-d) δ ppm = 2.30 (s, 1H), 3.00 - 3.16 (m, 1H), 3.34 - 3.50 (m, 1 H), 4.51 (dd, 1 H), 5.08 - 5.27 (m, 1 H), 7.22 - 7.39 (m, 4H).
MS (CI): [M + H]⁺ = 192.
[α]_{D} (*c* = 1.00, CHCl₃) = +49.3°

### Example 3: (1S,2S)-2-[¹⁸F]fluoro-N-[(1,1-²H₂)prop-2-yn-1-yl]indan-1-amine

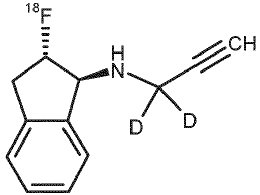

Aqueous [¹⁸F]Fluoride (21.5 GBq) was trapped on a Sep-Pak light QMA cartridge (Waters) (activated with 5 mL 0.5M K₂CO₃ solution, 10 mL water and 10mL air) and eluted with 2 mL K₂₂₂/K₂CO₃-solution (5 mg K₂₂₂ in 0.95 mL acetonitrile, 1 mg K₂CO₃ in 0.05 mL water) into the reactor. The solvent was removed by heating at 80°C for 3 min (N₂ stream and vacuum) and at 120 °C for additional 3 min (vacuum). Anhydrous acetonitrile (1 mL) was added and evaporated as before. A solution of precursor **example 1** (5 mg) in 500 µL anhydrous dimethyl sulfoxide (DMSO) was added. After heating the reaction mixture at 130 °C for 5 min additional 0.5 mL 1 M HCl were added and the mixture was heated for 10 min at 100°C. The crude reaction mixture was then cooled down to room temperature and diluted with 4 mL mobile phase and purified by preparative HPLC: Synergi 4 µ Hydro-RP 80A; 250 x 10.00 mm 4 micron; isocratic, 85% water (0.1% TFA), 15% acetonitrile (0.1%TFA), flow: 4 mL/min; t_{R}∼18 min. The collected HPLC fraction was diluted with 40 mL water and immobilized on a Sep-Pak C18 plus cartridge (Waters), which was washed with 5 mL water and eluted with 1 mL ethanol to deliver the 4.0 GBq of the ¹⁸F labelled product (28 % rc. yield, corrected for decay; >99% HPLC) in 1000 µl ethanol in a overall synthesis time of ∼70 min. The desired ¹⁸F labelled product of **example 3** (t_{R}=2.26 min) was analyzed using analytical HPLC: ACE3-C18 50 mm x 4,6 mm; solvent gradient: start 5 % acetonitrile - 95 % acetonitrile in 0.1 % trifluoroacetic acid in 7 min., flow: 2 mL/min and confirmed by co-injection with the corresponding non-radioactive ¹⁹F-fluoro-standard of **example 2** on the analytical HPLC (t_{R}=2.20 min).

### Examples demonstrating the superiority of the compounds according to the invention

### Specificity

Binding of the [¹⁸F]rasagiline (compound of formula F) was investigated on human brain sections from normal subjects using a standard protocol.

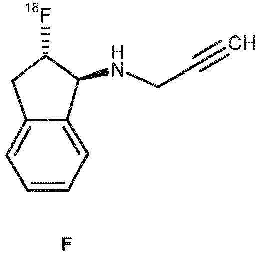

In brief, whole brain hemispheres were cut at a 100 µm thickness in a Cryocut (Leica, Germany), thaw mounted onto gelatine glass slides and kept at -25°C before use. Thereafter, the slides containing the regions of interest (e.g. hippocampus, thalamus) were removed and brought to room temperature. The sections were incubated with 0.2 MBq/mL [¹⁸F] compound **F** in 50 mM TRIS / 0.1% BSA (pH 7.4) with salts (120 mM NaCl, 5 mM KCl, 1 mM CaCl₂, 1 mM MgCl₂) for 90 min at room temperature and washed three times for 5 min each in 50 mM TRIS (pH 7.4). The sections were dipped once into ice cold distilled water, dried at room temperature and exposed to Phosphorlmager plates (FUJI BAS 5000) overnight.

For detection of the specificity of the signals an excess (10 µM) of deprenyl, rasagiline (both for MAO-B) and pirlindole (for MAO-A), respectively, was used together with the ¹⁸F labelled rasagiline (compound **F**).

Autoradiography using [¹⁸F]rasagiline on human brain slices from a healthy subject resulted in.the detection of a high signal intensity in the globus pallidus, the putamen, caudate, thalamus and hippocampus, regions with known MAO-B activity. The signals could not be blocked by an excess of pirlindole, a reversible MAO-A inhibitor (Pharmacol. Res. 1997, 36: 23-33), in the incubation solution. An excess of deprenyl or rasagiline, respectively, added to the incubation solution completely blocked the [¹⁸F]rasagiline (compound **F**) signal. These data show that [¹⁸F]rasagiline is a ligand specifically interacting with MAO-B. In summary, [¹⁸F]rasagiline (compound **F**) labelled specifically brain structures known to express MAO-B such as the hippocampus, caudate, putamen and thalamus.

These signals were blocked by an excess of compounds specific for MAO-B but not by pirlindole, a MAO-A specific compound.

### Uptake and wash-out

Thus, demonstrating the specificity of the signals, [¹⁸F]rasagiline (compound **F**) has been tested in a cynomolgus monkey. One monkey weighing 6.3 kg was injected i.v. with 163 MBq [¹⁸F]rasagiline (compound **F**) under sevofluorane anaesthesia. In a HRRT camera (Siemens Molecular Imaging) brain time activity curves (TAC) have been monitored and the standard uptake values (SUV) as shown in Fig. 1 were calculated. The initial brain uptake was ca. 280 %SUV within whole brain (Fig. 1, left panel) and ca. 400 %SUV when a striatal (caudate and putamen) and thalamic region of interest (ROI) were investigated (Fig. 1, right panel). The TACs for striatum and thalamus levelled at a comparable value, as did the TACs for cortex and cerebellum but the latter one at a lower value (Fig. 1, right panel).

In a second step, [¹⁸F]rasagiline has been synthesized as the di-deuterated version [¹⁸F],(²H₂)rasagiline (compound **1**).

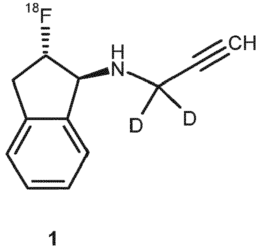

Two cynomolgus monkeys weighting 5.25 and 6.2 kg, respectively were investigated. They were injected i.v. under sevofluorane anaesthesia with 168 MBq and 174 MBq [¹⁸F], (²H₂)rasagiline (**1**), respectively. In a HRRT camera (Siemens Molecular Imaging) brain time activity curves (TAC) have been monitored and standard uptake values (SUV) as shown in Fig. 2 were calculated. It was found, that the initial uptake of [¹⁸F], (²H₂)rasagiline (**1**) in whole brain was comparable to that of [¹⁸F]rasagiline (compound **F**). The same was observed for the brain regions analyzed, i.e. striatum, thalamus, cortex and cerebellum.However, the TACs had now a shape characterized by a peak immediately after injection and elimination until a plateau is reached (steady state), as it is usually observed for reversible ligands (Fig. 2). [¹⁸F]rasagiline PET images from a specific plane of the monkey brain, covering striatum and thalamus as regions with high MAO B activity, demonstrated a high and specific signal in these regions whereas the signal in cerebral cortical structures was low. When using [¹⁸F], (²H₂)rasagiline for detection of MAO B activity in brain regions with high MAO B activity - striatum and thalamus - the signal declined to a lower value. It was still prominent and well distinguishable from the surprising low signal in cerebral cortical areas being in line with the %SUV TACs at steady state.

To demonstrate specificity of [¹⁸F], (²H₂)rasagiline (**1**) one monkey was pre-treated with 0.5 mg/kg (L)-deprenyl, a known irreversible inhibitor of MAO-B. This pre-treatment led to a strong decrease in uptake by 40 to 47%, respectively, in the investigated ROIs (Striatum 45%, Thalamus 47%, Cerebellum 46% and cortex 40%) demonstrating specificity of the [¹⁸F], (²H₂)rasagiline (Fig. 3).

### Metabolic profile

In addition, the blood radioactivity profiles have been monitored over time using radio-HPLC measurements and were decay corrected (d.c.) (Fig. 4 and 5). For [¹⁸F]rasagiline (compound F) eight ¹⁸F labelled metabolites have been observed in addition to the parent compound in plasma over the time period of the investigation (Fig. 4, Table 1).

Unexpectedly, there was a strong reduction in the number of metabolites from eight to two when the blood radioactivity profiles of [¹⁸F], (²H₂)rasagiline (**1**) were monitored (Fig. 5, Table 2).

In agreement with this, the metabolite corrected blood input curve from the PET experiments with [¹⁸F], (²H₂)rasagiline (**1**) showed an increased blood radioactivity over time when the curve was compared to the respective curve of the non-deuterated compound (Fig. 6).

### Reduced trapping in steady state phase

A particularly important improvement of MAO-B imaging is the surprising technical effect that a decrease in signal intensity from [¹⁸F]rasagiline (compound **F**) towards [¹⁸F], (²H₂)rasagiline (**1**) is between 2.4 -3 times in the brain regions investigated during the steady state phase (see Fig. 7). The reduced trapping rate of deuterated [¹⁸F], (²H₂)rasagiline (compound **1**) compared to [¹⁸F] rasagiline (compound **F**) enables an improved quantification of MAO-B activity and avoids blood flow limitation of delivery of the tracer, since it is known that a high trapping rate is responsible for an underestimation of the MAO-B signal in regions with high MAO-B activity (Fowler et al. Mol. Imaging Biol. 2005, 7: 377-387).

The effect strongly exceeds effects reported for compounds from the prior art and hence could not be expected by the person skilled in the art.

From studies using [¹¹C]deprenyl (compound **H**) it is known that the MAO-B signal is underestimated in regions with high MAO-B activity due to high trapping rate that is similar to or exceeds delivery (Fowler et al. J Nucl Med 1995; 36: 1255).

Putting deuteron atoms in place at [¹¹C]deprenyl (compound **H**) yielding [¹¹C], (²H₂)deprenyl (compound **G**) has been reported to result in a reduced trapping rate.

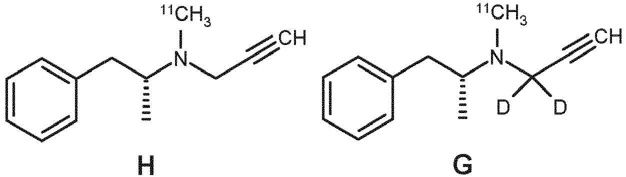

This leads to a more reliable quantification of the signal. But the positive effect of deuteration on the decrease in signal intensity for [¹¹C], (²H₂)deprenyl (compound G) is only 1.2 - 2.0-fold being observed in healthy baboon and human brain regions (e.g. striatum, thalamus, cortex, Fowler et al. J. Neurochem 1988, 51: 1524-1534; J. Nucl. Med. 1995, 36: 1255-1262; Mol. Imaging Biol. 2005, 7: 377-387).

Thus, the aforementioned improved ratio of signal intensity of [¹⁸F]rasagiline (compound **F**) to [¹⁸F], (²H₂)rasagiline (**1**) is surprisingly higher (2.4 to 3) than the published ratio of signal intensity of [¹¹C]deprenyl (compound **H**) to [¹¹C], (²H₂)deprenyl (compound **G**) (∼1.2 to 2.0) (Fig. 8).

More specifically, the unexpected observation is that regions with high MAO-B expression had a more pronounced decrease in signal intensity when compared to the effect that was expected from data published for [¹¹C], (²H₂)deprenyl (compound **G**) and [¹¹C]deprenyl (compound **H**).

In striatum the decrease was 2.9 times for [¹⁸F, ²H₂]rasagiline (**1**) compared to only 1.7 times published for [¹¹C], (²H₂)deprenyl (compound **G**). Furthermore, in thalamus the decrease was 2.7 times for [¹⁸F], (²H₂)rasagiline (**1**) compared to only 1.3 times published for [¹¹C], (²H₂)deprenyl (compound **G**).

Another unexpected finding was in the cerebellum, an expected reference region with very low MAO-B activity. Here the decrease in signal intensity was 3.0 and thus, being twice as much as published for [¹¹C], (²H₂)deprenyl levelling at 1.5.

Specifically, its low signal in the reference region cerebellum renders the compounds of the invention as superior PET imaging agents leading to increased ratios of target regions to cerebellum as a reference region.

**Table 1**

| Metabolite no. | **M1** | **M2** | **M3** | **M4** | **M5** | **M6** | **M7** | **M8** | **Parent d.c.** |
|---|---|---|---|---|---|---|---|---|---|
| Retention time rt [min] | 2,8 | 4,6 | 6,1 | 9,4 | 11,6 | 12,8 | 13,5 | 14,21 | |

| sample time [min] | **Radio-HPLC [area%]** | | | | | | | | |
|---|---|---|---|---|---|---|---|---|---|
| 5,23 | 1,422 | | 0,826 | 9,163 | | 1,547 | | | 87,042 |
| 16,68 | 15,273 | 3,575 | | 6,18 | | | 2,71 | 1,294 | 70,968 |
| 31,67 | 21,345 | | 11,422 | 7,62 | 1,488 | | | | 58,125 |
| 47,43 | 23,246 | | 18,876 | 4,325 | 1,092 | | | 1,638 | 50,823 |
| 62,67 | 28,757 | | 21,024 | 6,057 | 2,236 | | 4,081 | | 37,845 |
| 77,52 | 32,1 | | 22,249 | 4,565 | | | 5,285 | | 35,801 |
| 92,40 | 31,134 | | 29,414 | 6,326 | 1,154 | | | | 31,971 |
| 121,87 | 51,939 | | 26,806 | 1,738 | | | | | 19,517 |

**Table 2**

| Metabolite no. | **M1** | **M2** | **Parent d.c.** |
|---|---|---|---|
| Retention time rt[min] | 2,9 | 6,63 | |

| sample time [min] | **Radio-HPLC [area%]** | | |
|---|---|---|---|
| 4,35 | 4,009 | | 95,991 |
| 17,13 | 16,318 | 8,262 | 75,420 |
| 33,15 | 24,358 | 14,355 | 61,287 |
| 48,73 | 27,659 | 16,914 | 55,426 |
| 61,63 | 31,262 | 19,756 | 48,981 |
| 78,22 | 34,379 | 20,885 | 44,736 |
| 91,98 | 38,222 | 22,991 | 38,787 |
| 116,95 | 38,759 | 27,347 | 33,894 |

## Claims

1. A compound of formula I 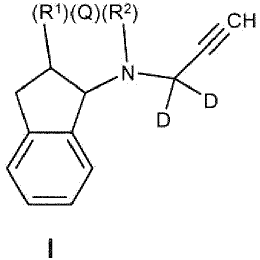 wherein
(R¹)(Q)(R²) is selected from the group consisting of
(O)(bond)(S(O)₂),
([¹⁸F]fluoro)(blank)(S(O)₂(O⁻)(X⁺)),
(F)(blank)(S(O)₂(O⁻)(X⁺)),
([¹⁸F]fluoro)(blank)(H), and
(F)(blank)(H);
X⁺ is selected from the group consisting of Li⁺, Na⁺, K⁺, Cs⁺, and/or Rb⁺;
bond means that the atoms or atom groups adjacent to the bond share at least one pair of electrons and thus are chemically bonded to each other;
blank means that there is neither a chemical bond nor an atom or an atom group connecting R¹ and R².
including all isomeric forms of said compound, including but not limited to enantiomers and diastereoisomers as well as mixtures of isomers, and any pharmaceutically acceptable salt thereof.

2. A compound of claim 1, **characterized in that** the compound is selected from a compound of formula **Ia** 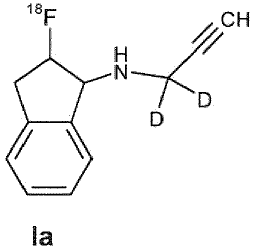 including all isomeric forms of said compound, including but not limited to enantiomers and diastereoisomers as well as mixtures of isomers, and any pharmaceutically acceptable salt thereof.

3. A compound of claim 2, **characterized in that** the compound is (1*S*,2*S*)-2-[¹⁸F]fluoro-*N*-[(1,1-²H₂)prop-2-yn-1-yl]indan-1-amine 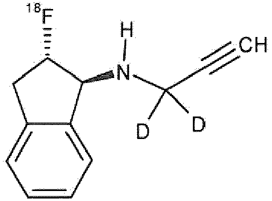 including any pharmaceutically acceptable salt thereof.

4. A compound of claim 1, **characterized in that** the compound is selected from a compound of formula **Ib** 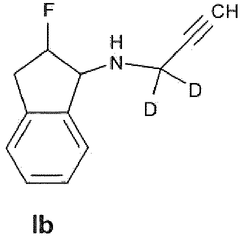 including all isomeric forms of said compound, including but not limited to enantiomers and diastereoisomers as well as mixtures of isomers, and any pharmaceutically acceptable salt thereof.

5. A compound of claim 4, **characterized in that** the compound is (1*S*,2*S*)-2-fluoro-*N-*[(1,1-²H₂)prop-2-yn-1-yl]indan-1-amine 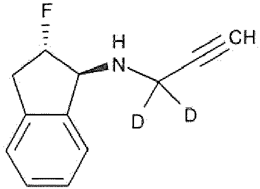 including any pharmaceutically acceptable salt thereof.

6. A compound of claim 1, **characterized in that** the compound is selected from a compound of formula **Ic** 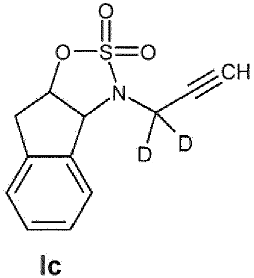 including all isomeric forms of said compound, including but not limited to enantiomers and diastereoisomers as well as mixtures of isomers, and any pharmaceutically acceptable salt thereof.

7. A compound of claim 6, **characterized in that** the compound is (3a*S*,8a*R*)-3-[(1,1-²H₂)prop-2-yn-1-yl]-3,3a,8,8a-tetrahydroindeno[1,2*d*][1,2,3] oxathiazole 2,2-dioxide 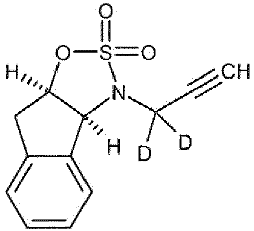 including any pharmaceutically acceptable salt thereof.

8. A compound of claim 1, **characterized in that** the compound is selected from a compound of formula **Id** 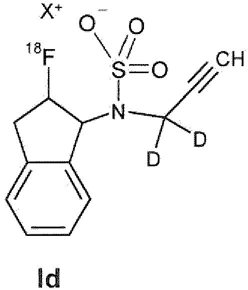 wherein X⁺ is selected from the group Li⁺, Na⁺, K⁺, Cs⁺, and/or Rb⁺;
including all isomeric forms of said compound, including but not limited to enantiomers and diastereoisomers as well as mixtures of isomers, and any pharmaceutically acceptable salt thereof.

9. A compound of claim 8, **characterized in that** the compound is potassium *N*-[(1*S*,2*S*)-2-[¹⁸F]fluoroindan-1-yl]-*N*-[(1,1-²H₂)prop-2-yn-1-yl]sulfamate 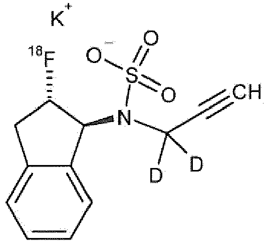 including any pharmaceutically acceptable salt thereof.

10. A compound of claim 1, **characterized in that** the compound is selected from a compound of formula **Ie** 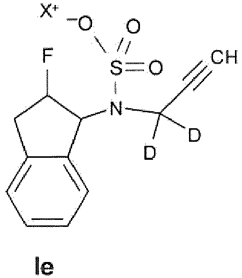 wherein X⁺ is selected from the group Li⁺, Na⁺, K⁺, Cs⁺, and/or Rb⁺; including all isomeric forms of said compound, including but not limited to enantiomers and diastereoisomers as well as mixtures of isomers, and any pharmaceutically acceptable salt thereof.

11. A compound of claim 10, **characterized in that** the compound is caesium *N*-[(1*S*,2*S*)-2-fluoroindan-1-yl]-*N*-[(1,1-²H₂)prop-2-yn-1-yl]sulfamate 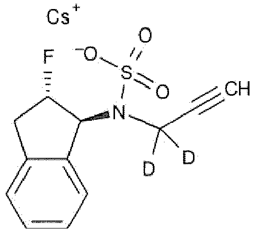 including any pharmaceutically acceptable salt thereof.

12. A compound of claims 2 or 3 for use as a diagnostic compound for PET imaging.

13. A diagnostic composition comprising a compound of claims 2 or 3.

14. A compound of claims 2 or 3 for use as a diagnostic compound for PET imaging of CNS diseases.

15. A diagnostic composition comprising a compound of claims 2 or 3 for use in PET imaging of CNS diseases.

16. A compound or a composition according to claims 12 to 15 for use in imaging of Alzheimer's disease.

17. Method for the synthesis of a compound of according to formula I 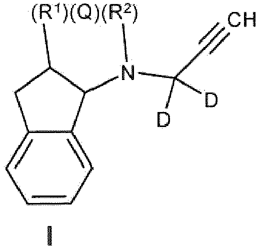 wherein
(R¹)(Q)(R²) is selected from the group consisting of
(O)(bond)(S(O)₂),
([¹⁸F]fluoro)(blank)(S(O)₂(O⁻)(X⁺)),
(F)(blank)(S(O)₂(O⁻)(X⁺)),
([¹⁸F]fluoro)(blank)(H), and
(F)(blank)(H);
X⁺ is selected from the group consisting of Li⁺, Na⁺, K⁺, Cs⁺, and/or Rb⁺; bond means that the atoms or atom groups adjacent to the bond share at least one pair of electrons and thus are chemically bonded to each other;
blank means that there is neither a chemical bond nor an atom or an atom group connecting R¹ and R²;
the method comprising the steps
- converting a compound of formula III 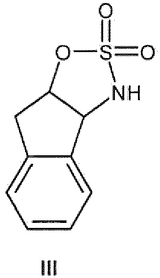 into a compound of formula IV 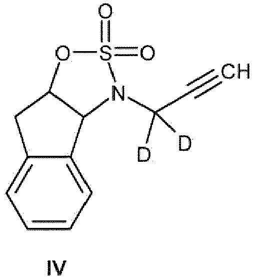
- optionally reacting obtained compound of formula IV with a ¹⁸F- or ¹⁹F-fluorination agent, thereby obtaining a compound of formula **Id** or **Ie**, 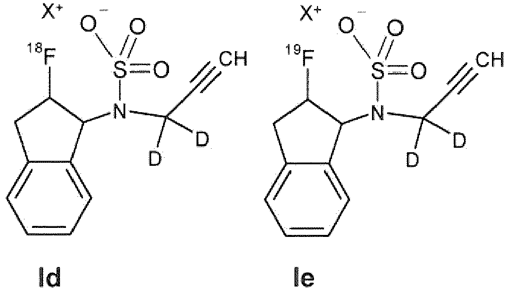 wherein X⁺ is selected from the group consisting of Li⁺, Na⁺, K⁺, Cs⁺, and/or Rb⁺,
- optionally reacting obtained compound of formula **Ie** or **Id** with acid, thereby obtaining a compound of formula **Ia** or **Ib** 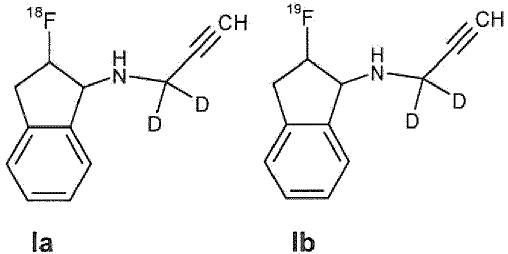
- optionally converting obtained compound **Ia, Ib, Ic, Id** or **Ie** into a suitable salt of inorganic or organic bases, hydrates, and/or solvates thereof.

18. A kit comprising at least one sealed container comprising a compound or a composition according to claims 1-16.

## Patentansprüche

1. Verbindung der Formel I, 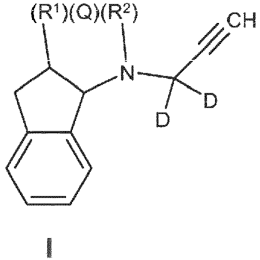 wobei
(R¹)(Q)(R²) ausgewählt ist aus der Gruppe bestehend aus (O)(Bindung)(S(O)₂),
([¹⁸F]Fluor)(ungebunden)(S(O)₂(O⁻)(X⁺)),
(F)(ungebunden)(S(O)₂(O⁻)(X⁺)),
([¹⁸F]Fluor)(ungebunden)(H), und
(F)(ungebunden)(H);
X⁺ ausgewählt ist aus der Gruppe bestehend aus Li⁺, Na⁺, K⁺, Cs⁺ und/oder Rb⁺;
Bindung bedeutet, dass die an der Bindung anliegenden Atome oder Atomgruppen mindestens ein Elektronenpaar gemeinsam haben und somit chemisch aneinander gebunden sind;
ungebunden bedeutet, dass weder eine chemische Bindung noch ein Atom oder eine Atomgruppe vorliegt, die R¹ und R² verbindet.
einschließlich aller isomeren Formen der genannten Verbindung, einschließlich, aber nicht beschränkt auf Enantiomere und Diastereoisomere sowie Gemische von isomeren, und are pharmazeutisch verträglichen Salze davon.

2. Verbindung nach Anspruch 1, **dadurch gekennzeichnet, dass** die Verbindung aus einer Verbindung der Formel **Ia** ausgewählt ist 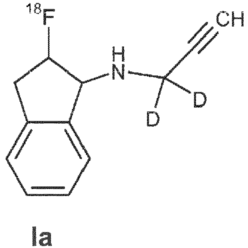 einschließlich aller isomeren Formen der genannten Verbindung, einschließlich, aber nicht beschränkt auf Enantiomere und Diastereoisomere sowie Gemische von Isomeren, und alle pharmazeutisch verträglichen Salze davon.

3. Verbindung nach Anspruch 2, **dadurch gekennzeichnet, dass** die Verbindung (1*S*,2*S*)-2-[¹⁸F]Fluor-*N*-[(1,1-²H₂)prop-2-yn-1-yl]indan-1-amin ist, 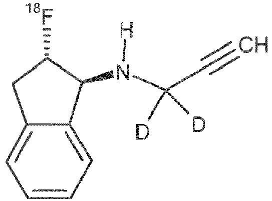 einschließlich aller pharmazeutisch verträglichen Salze davon.

4. Verbindung nach Anspruch 1, **dadurch gekennzeichnet, dass** die Verbindung aus einer Verbindung der Formel **Ib** ausgewählt ist, 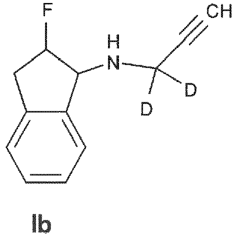 einschließlich aller isomeren Formen der genannten Verbindung, einschließlich, aber nicht beschränkt auf Enantiomere und Diastereoisomere sowie Gemische von Isomeren, und alle pharmazeutisch verträglichen Salze davon.

5. Verbindung nach Anspruch 4, **dadurch gekennzeichnet, dass** die Verbindung (1*S*,2*S*)-2-Fluor-*N*-[(1,1-²H₂)prop-2-yn-1-yl]indan-1-amin ist, 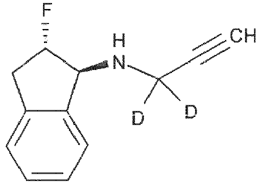 einschließlich aller pharmazeutisch verträglichen Salze davon.

6. Verbindung nach Anspruch 1, **dadurch gekennzeichnet, dass** die Verbindung aus einer Verbindung der Formel **Ic** ausgewählt ist, 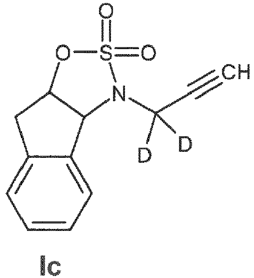 einschließlich aller isomeren Formen der genannten Verbindung, einschließlich, aber nicht beschränkt auf Enantiomere und Diastereoisomere sowie Gemische von Isomeren, und alle pharmazeutisch verträglichen Salze davon.

7. Verbindung nach Anspruch 6, **dadurch gekennzeichnet, dass** die Verbindung (3a*S*,8a*R*)-3-[(1,1-²H₂)Prop-2-yn-1-yl]-3,3a,8,8a-tetrahydroindeno[1,2*d*][1,2,3] oxathiazol 2,2-dioxid ist, 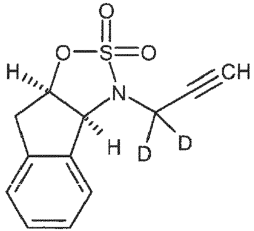 einschließlich aller pharmazeutisch verträglichen Salze davon.

8. Verbindung nach Anspruch 1, **dadurch gekennzeichnet, dass** die Verbindung aus einer Verbindung der Formel **Id** ausgewählt ist, 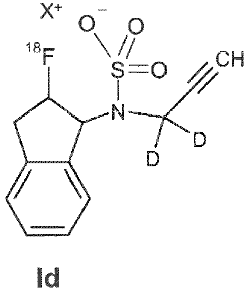 wobei X⁺ ausgewählt ist aus der Gruppe bestehend aus Li⁺, Na⁺, K⁺, Cs⁺ und/oder Rb⁺;
einschließlich aller isomeren Formen der genannten Verbindung, einschließlich, aber nicht beschränkt auf Enantiomere und Diastereoisomere sowie Gemische von Isomeren, und alle pharmazeutisch verträglichen Salze davon.

9. Verbindung nach Anspruch 8, **dadurch gekennzeichnet, dass** die Verbindung Kalium *N*-[(1*S*,2*S*)-2-[¹⁸F]fluorindan-1-yl]-*N*-[(1,1-²H₂)prop-2-yn-1-yl]sulfamat ist, 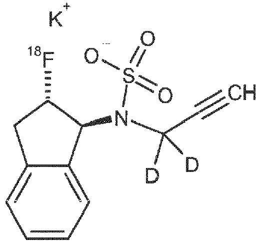 einschließlich aller pharmazeutisch verträglichen Salze davon.

10. Verbindung nach Anspruch 1, **dadurch gekennzeichnet, dass** die Verbindung aus einer Verbindung der Formel **Ie** ausgewählt ist, 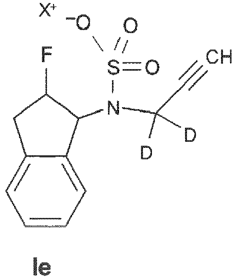 wobei X⁺ ausgewählt ist aus der Gruppe bestehend aus Li⁺, Na⁺, K⁺, Cs⁺ und/oder Rb⁺;
einschließlich aller isomeren Formen der genannten Verbindung, einschließlich, aber nicht beschränkt auf Enantiomere und Diastereoisomere sowie Gemische von Isomeren, und alle pharmazeutisch verträglichen Salze davon.

11. Verbindung nach Anspruch 10, **dadurch gekennzeichnet, dass** die Verbindung Caesium *N*-[(1*S*,2*S*)-2-fluorindan-1-yl]-*N*-[(1,1-²H₂)prop-2-yn-1-yl]sulfamat ist, 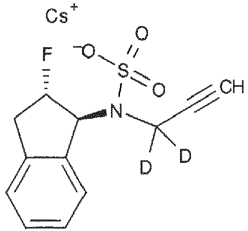 einschließlich aller pharmazeutisch verträglichen Salze davon.

12. Verbindung nach Anspruch 2 oder 3 zur Verwendung als diagnostische Verbindung für die Positronen-Emissions-Tomographie (PET-Bildgebung).

13. Diagnostische Zusammensetzung umfassend eine Verbindung nach Anspruch 2 oder 3.

14. Verbindung nach Anspruch 2 oder 3 zur Verwendung als diagnostische Verbindung bei der PET-Bildgebung von ZNS-Erkrankungen.

15. Diagnostische Zusammensetzung umfassend eine Verbindung nach Anspruch 2 oder 3 zur Verwendung bei der PET-Bildgebung von ZNS-Erkrankungen.

16. Verbindung oder Zusammensetzung nach den Ansprüchen 12 bis 15 zur Verwendung bei der Bildgebung der Alzheimererkrankung.

17. Verfahren für die Synthese einer Verbindung nach Formel **I** 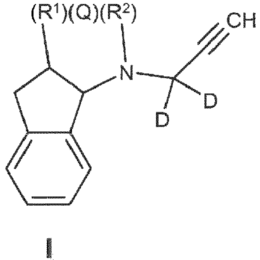 wobei
(R¹)(Q)(R²) ausgewählt ist aus der Gruppe bestehend aus (O)(Bindung)(S(O)₂),
([¹⁸F]Fluor)(ungebunden)(S(O)₂(O⁻)(X⁺)),
(F)(ungebunden)(S(O)₂(O⁻)(X⁺)),
([¹⁸F]Fluor)(ungebunden)(H), und
(F)(ungebunden)(H);
X⁺ ausgewählt ist aus der Gruppe bestehend aus Li⁺, Na⁺, K⁺, Cs⁺ und/oder Rb⁺; Bindung bedeutet, dass die an der Bindung anliegenden Atome oder Atomgruppen mindestens ein Elektronenpaar gemeinsam haben und somit chemisch aneinander gebunden sind;
ungebunden bedeutet, dass weder eine chemische Bindung noch ein Atom oder eine Atomgruppe vorliegt, die R¹ und R² verbindet;
das Verfahren umfasst die folgenden Schritte:
- Umwandlung einer Verbindung der Formel III 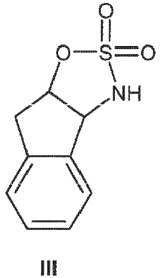 in eine Verbindung der Formel IV 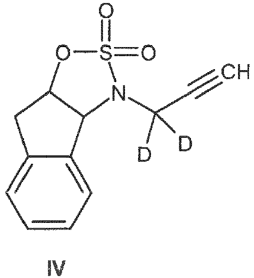
- optionale Reaktion der mit Formen IV erhaltenen Verbindung mit einem ¹⁸F- bzw. ¹⁹F-Fluorierungsmittei und dadurch Erhalt einer Verbindung der Formel **Id** oder **Ie**, 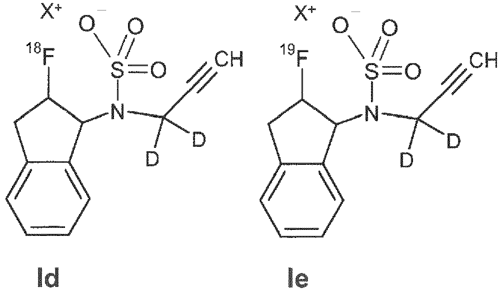 wobei X⁺ ausgewählt ist aus der Gruppe bestehend aus Li⁺, Na⁺, K⁺, Cs⁺ und/oder Rb⁺;
- optionale Reaktion der mit Formel **Ie** oder **Id** erhaltenen Verbindung mit Säure und dadurch Erhalt einer Verbindung der Formel **Ia** oder **Ib** 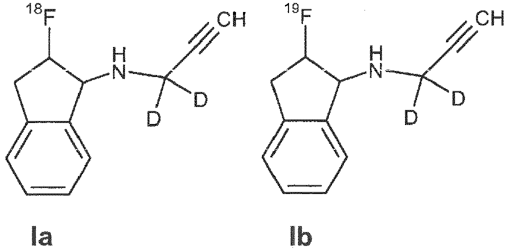
- optionale Umwandlung der erhaltenen Verbindung **Ia, Ib**, **Ic**, **Id** oder **Ie** in ein geeignetes Salz von anorganischen oder organischen Basen, Hydraten und/oder Solvaten davon.

18. Kit umfassend mindestens einen versiegelten Behälter, der eine Verbindung oder eine Zusammensetzung nach den Ansprüchen 1-16 umfasst.

## Revendications

1. Composé de la formule I 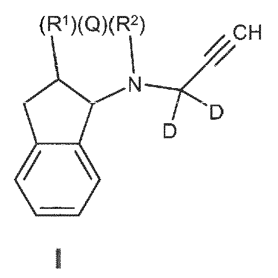 dans laquelle
(R¹)(Q)(R²) est choisi parmi le groupe constitué par
(O)(liaison)(S(O)₂),
([¹⁸F]fluoro)(non lié)(S(O)₂(O⁻)(X⁺)),
(F)(non lié)(S(O)₂(O⁻)(X⁺)),
([¹⁸F]fluoro)(non lié)(H), et
(F)(non lié)(H);
X⁺ est choisi parmi le groupe constitué par Li⁺, Na⁺, K⁺, Cs⁺, et/ou Rb⁺;
liaison signifie que les atomes ou les groupes d'atomes à proximité de la liaison partagent au moins une paire d'électrons et sont ainsi liés chimiquement les uns aux autres;
non lié signifie qu'il n'y a ni liaison chimique ni atome ou groupe d'atomes reliant R¹ et R².
y compris toutes les formes isomériques dudit composé, y compris, sans s'y limiter, les énantiomères et les diastéréoisomères ainsi que les mélanges d'isomères, et un quelconque sel pharmaceutiquement acceptable de ceux-ci.

2. Composé de la revendication 1, **caractérisé en ce que** le composé est choisi parmi un composé de la formule **Ia** 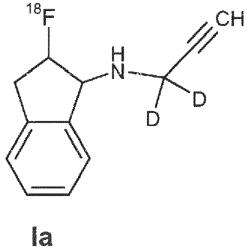 y compris toutes les formes isomériques dudit composé, y compris, sans s'y limiter, les énantiomères et les diastéréoisomères ainsi que les mélanges d'isomères, et un quelconque sel pharmaceutiquement acceptable de ceux-ci.

3. Composé de la revendication 2, **caractérisé en ce que** le composé est (1S,2S)-2-[¹⁸F]fluoro-*N*-[(1,1-²H₂)prop-2-yn-1-yl]indan-1-amine 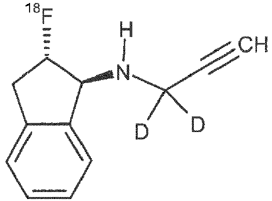 y compris un quelconque sel pharmaceutiquement acceptable de celui-ci.

4. Composé de la revendication 1, **caractérisé en ce que** le composé est choisi parmi un composé de la formule **Ib** 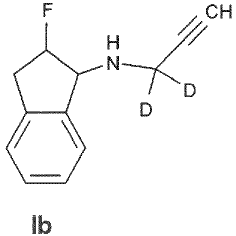 y compris toutes les formes isomériques dudit composé, y compris, sans s'y limiter, les énantiomères et les diastéréoisomères ainsi que les mélanges d'isomères, et un quelconque sel pharmaceutiquement acceptable de ceux-ci.

5. Composé de la revendication 4, **caractérisé en ce que** le composé est (1S,2S)-2-fluoro-*N*-[(1,1-²H₂)prop-2-yn-1-yl]indan-1-amine 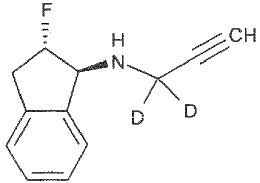 y compris un quelconque sel pharmaceutiquement acceptable de celui-ci.

6. Composé de la revendication 1, **caractérisé en ce que** le composé est choisi parmi un composé de la formule **Ic** 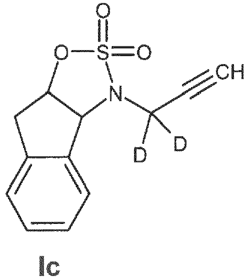 y compris toutes les formes isomériques dudit composé, y compris, sans s'y limiter, les énantiomères et les diastéréoisomères ainsi que les mélanges d'isomères, et un quelconque sel pharmaceutiquement acceptable de ceux-ci.

7. Composé de la revendication 6, **caractérisé en ce que** le composé est (3a*S*,8a*R*)-3-[(1,1-²H₂)prop-2-yn-1-yl]-3,3a,8,8a-tétrahydroindéno[1,2*d*][1,2,3]oxathiazole 2,2-dioxyde 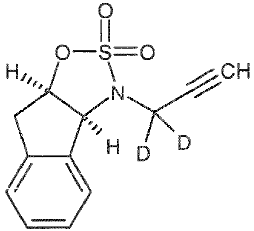 y compris un quelconque sel pharmaceutiquement acceptable de celui-ci.

8. Composé de la revendication 1, **caractérisé en ce que** le composé est choisi parmi un composé de la formule **Id** 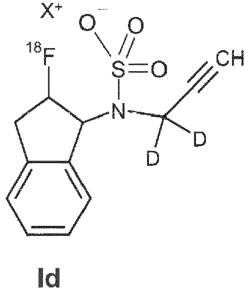 dans laquelle X⁺ est choisi parmi le groupe Li⁺, Na⁺, K⁺, Cs⁺, et/ou Rb⁺;
y compris toutes les formes isomériques dudit composé, y compris, sans s'y limiter, les énantiomères et les diastéréoisomères ainsi que les mélanges d'isomères, et un quelconque sel pharmaceutiquement acceptable de ceux-ci.

9. Composé de la revendication 8, **caractérisé en ce que** le composé est potassium *N*-[(1S,2S)-2-[¹⁸F]fluoroindan-1-yl]-N-[(1,1-²H₂)prop-2-yn-1-yl]sulfamate 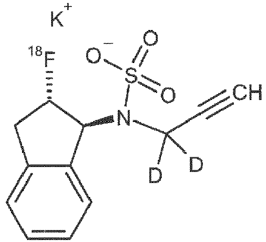 y compris un quelconque sel pharmaceutiquement acceptable de celui-ci.

10. Composé de la revendication 1, **caractérisé en ce que** le composé est choisi parmi un composé de la formule **Ie** 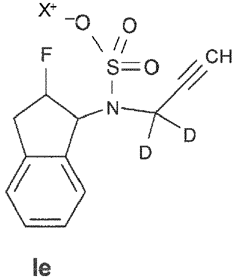 dans laquelle X⁺ est choisi parmi le groupe Li⁺, Na⁺, K⁺, Cs⁺, et/ou Rb⁺; y compris toutes les formes isomériques dudit composé, y compris, sans s'y limiter, les énantiomères et les diastéréoisomères ainsi que les mélanges d'isomères, et un quelconque sel pharmaceutiquement acceptable de ceux-ci.

11. Composé de la revendication 10, **caractérisé en ce que** le composé est césium *N*-[(1S,2S)-2-fluoroindan-1-yl]-N-[(1,1-²H₂)prop-2-yn-1-yl]sulfamate 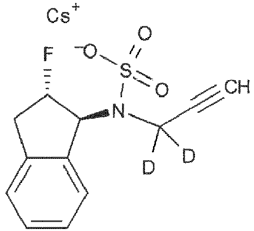 y compris un quelconque sel pharmaceutiquement acceptable de celui-ci.

12. Composé des revendications 2 ou 3 destiné à être utilisé en tant que composé diagnostique pour l'imagerie TEP.

13. Composition diagnostique comprenant un composé des revendications 2 ou 3.

14. Composé des revendications 2 ou 3 destiné à être utilisé en tant que composé diagnostique pour l'imagerie TEP des maladies du SNC.

15. Composition diagnostique comprenant un composé des revendications 2 ou 3, destinée à être utilisé dans l'imagerie TEP des maladies du SNC.

16. Composé ou composition diagnostique selon les revendications 12 à 15 destiné à être utilisé dans l'imagerie de la maladie d'Alzheimer.

17. Méthode pour la synthèse d'un composé selon la formule **I** 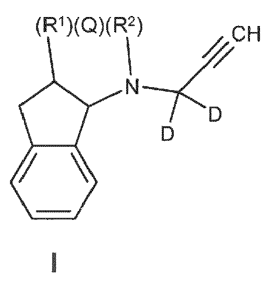 dans laquelle
(R¹)(Q)(R²) est choisi parmi le groupe constitué par
(O)(liaison)(S(O)₂),
([¹⁸F]fluoro)(non lié)(S(O)₂(O)⁻)(X⁺)),
(F)(non lié)(S(O)₂(O⁻)(X⁺)),
([¹⁸F]fluoro)(non lié)(H), et
(F)(non lié)(H);
X⁺ est choisi parmi le groupe constitué par Li⁺, Na⁺, K⁺, Cs⁺, et/ou Rb⁺; liaison signifie que les atomes ou les groupes d'atomes à proximité de la liaison partagent au moins une paire d'électrons et sont ainsi liés chimiquement les uns aux autres;
non lié signifie qu'il n'y a ni liaison chimique ni atome ou groupe d'atomes reliant R¹ et R².
le procédé consistant des étapes
- Conversion d'un composé de la formule **III** 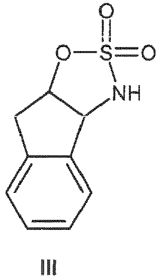 en composé de la formule **IV** 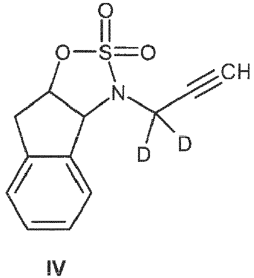
- réaction facultative du composé obtenu de la formule **IV** avec un agent de fluoration F¹⁸ ou F¹⁹, obtenant ainsi un composé de la formule **Id** ou **Ie**, 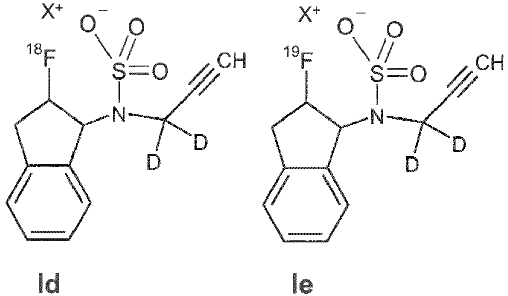 dans laquelle X⁺ est choisi parmi le groupe constitué par Li⁺, Na⁺, K⁺, Cs⁺, et/ou Rb⁺;
- réaction facultative du composé obtenu de la formule **Ie** ou **Id** avec l'acide, obtenant ainsi un composé de la formule **Ia** ou **Ib** 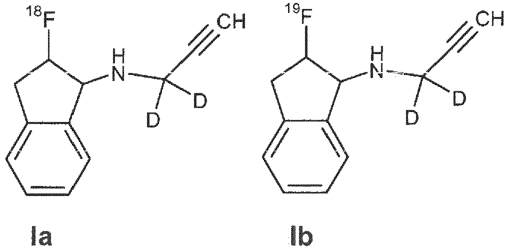
- conversion facultative du composé **Ia**, **Ib, Ic**, **Id** ou **Ie** obtenu en un sel de bases inorganiques ou organiques acceptables, en hydrates et/ou en solvates de ceux-ci.

18. Trousse comportant au moins un récipient scellé comprenant un composé ou une composition selon les revendications 1 à 16.
